# EUROPEAN PATENT APPLICATION

(11) **EP 4 825 975 A1**
(43) Date of publication of application: **21.10.2026**
(21) Application number: 24903802.7
(22) Date of filing: 13.12.2024
(51) Int. Cl.: C12P 19/26

(54) **FERMENTED PRODUCT AND METHOD FOR PRODUCING FERMENTED PRODUCT**

(30) Priority: 13.12.2023 WO PCT/JP2023/044738
(71) Applicant: Novozymes A/S, 2880 Bagsvaerd (DK)
(72) Inventor: FUKUMOTO Kazunari, Tokyo 100-8185 (JP); YOKOI Tomoya, Tokyo 100-8185 (JP); HAYASHIDA Chinami, Tokyo 100-8185 (JP); ISEKI Moe, Tokyo 164-0001 (JP)
(74) Representative: NVS EPO Representatives
(86) International application number: PCT/JP2024/044306
(87) International publication number: WO 2025/127149

(57) **Abstract**

The present invention relates to a fermented product containing component X, component Y, and component Z, wherein component X is a sialic acid or a sialylated oligosaccharide, the content of component X per unit of solid content of the fermented product is 80 mass% or more, component Y is acetic acid, the ratio (content of component Y / content of component X) of the content of component Y relative to the content of component X in the fermented product is 0.010 or less on a mass basis, component Z is at least one of component Z1 and component Z2, and component Z is a component other than component X, and component Z1 is a neutral sugar and component Z2 is an acidic sugar.

## Description

### Technical Field

The present invention relates to a fermented product containing a sialic acid or sialylated oligosaccharide, and a method for producing the fermented product.

### BACKGROUND ART

Oligosaccharides contained in human breast milk (Human Milk Oligosaccharides, hereinafter referred to as "HMO") have attracted attention as prebiotic ingredients, and have been shown to be effective in promoting cognitive development, protecting against infections, and improving the intestinal environment in infants and young children (Non-Patent Document 1). Sialylated oligosaccharides are one of the important oligosaccharides contained in human breast milk, and are believed to possess physiological activities such as infection defense against viruses and bacteria, and the promotion of lactic acid bacteria proliferation. Furthermore, sialylated oligosaccharides are useful, for example, as products, raw materials, and intermediates for health foods, pharmaceuticals, and cosmetics. Sialic acids are also known as a component of breast milk. In particular, N-acetylneuraminic acid (hereinafter referred to as NeuAc) is a type of sialic acid, a general term for acidic amino sugars, and is widely used as a raw material for pharmaceuticals such as anti-influenza drugs, as well as in foods, cosmetics, and cell culture media.

To date, various production methods for obtaining high-purity sialylated oligosaccharides have been investigated. For example, a method is known in which 6'-sialyllactose [O-(N-acetyl-α-neuraminosyl)-(2→6)-O-β-D-galactopyranosyl-(1→4)-D-glucose] (hereinafter also referred to as "6SL"), produced by chemical synthesis, is spray-dried (Patent Document 1). Furthermore, a method for removing impurities by crystallizing the sodium salt of 6SL is known (Patent Document 2); however, since the addition of organic solvents such as methanol is necessary during the crystal precipitation process, there is a concern that it cannot be ruled out that these solvents remain in the final product, raising safety concerns. Therefore, in recent years, although the use of final products obtained by methods for purifying sialylated oligosaccharides produced by fermentation (Patent Documents 3 and 4) in foods and other products has become mainstream, it is difficult to remove the neutral sugars and organic acids generated during fermentation, and it is known that they remain as impurities to a certain extent. Furthermore, components derived from the fermentation bacteria that are mixed into the fermentation product also possess physical properties similar to those of sialic acids and sialylated oligosaccharides, and the removal of such contaminants is also required.

### Prior Art Documents

### Patent Documents

Patent Document 1: International Publication No. 2013/185780
Patent Document 2: International Publication No. 2017/086443
Patent Document 3: Japanese Unexamined Patent Application Publication (Translation of PCT Application) No. 2021-505170
Patent Document 4: Japanese Unexamined Patent Application Publication (Translation of PCT Application) No. 2020-531039

### Non-Patent Document

Non-Patent Document 1: Int J Pediatr., 04 August 2019; 2019:2390240.

### SUMMARY OF INVENTION

### TECHNICAL PROBLEM

In order to obtain a product containing high concentrations of sialylated oligosaccharides, it is necessary to reduce the incorporated amount of impurities, such as neutral sugars, acidic sugars other than sialylated oligosaccharides, and organic acids, in the final product. Methods for preventing such impurities from contaminating the final product include chemical synthesis and crystallization. However, these methods require the use of residual solvents, such as methanol, that are classified as Class 1 to Class 3, as defined in Guideline for Residual Solvents (Q3C) of the International Council for Harmonisation of Technical Requirements for Pharmaceuticals for Human Use (ICH), and it is necessary to strictly control these residual solvents to prevent their contamination of the final product. In particular, sialylated oligosaccharides are ingredients that are frequently used in products for infants and young children, and therefore require a high level of safety. Consequently, from a safety perspective, chemically synthesized products and crystals produced by the aforementioned methods present problems to be addressed in manufacturing methods for sialylated oligosaccharides.

Furthermore, regarding fermentation products produced by fermenting sialylated oligosaccharides and purified using ion exchange resins, etc., conventional methods inevitably result in the contamination of the final product with neutral sugars, such as lactose, which are present during the production process. Furthermore, for example, acetic acid, which falls under ICH-Q3C Class 3 and is generated during fermentation, is introduced into the final product as an impurity. Therefore, there has been a need to establish a production method that reduces these impurities and achieves a sufficient recovery rate of sialic acids or sialylated oligosaccharides in industrial production.

Accordingly, an object of the present invention is to provide a fermented product containing a sialic acid or sialylated oligosaccharide with a low impurity content, and a method for producing the fermented product.

### SOLUTION TO PROBLEM

As a result of diligent investigation into the above problem, the present inventors have found that it is possible to produce a fermented product which contains a sialic acid or sialylated oligosaccharide at a high content ratio and has a low impurity content using a method containing steps of treating solution A, which contains a sialic acid or sialylated oligosaccharide, with a strongly acidic cation exchange resin to obtain solution B, treating solution B with a weakly basic anion exchange resin to obtain solution C, adjusting the pH of solution C to obtain solution D, and treating solution D with a weakly basic anion exchange resin to obtain solution E.

More specifically, the present invention is as follows.
1. A fermented product containing components X, Y, and Z,
   wherein: component X is a sialic acid or a sialylated oligosaccharide;
   the content ratio of component X per unit of solid matter in the fermented product is 80 mass% or more; and
   component Y is acetic acid;
   the ratio (content of component Y / content of component X) of the content of component Y relative to the content of component X in the fermented product is 0.010 or less on a mass basis;
   component Z is at least one of component Z1 and component Z2, and component Z is a component other than component X; and
   component Z1 is a neutral sugar and component Z2 is an acidic sugar.
2. The fermented product according to 1 above, wherein component X is N-acetylneuraminic acid.
3. The fermented product according to 1 above, wherein component X is at least one compound selected from the group consisting of sialyllactose, sialyllacto-N-biose (SLNB), sialyllacto-N-acetylglucosamine (SLNac), sialyllacto-N-tetraose (LST), and disialyllacto-N-tetraose (DSLNT).
4. The fermented product according to 2 above, wherein component Z2 is at least one compound selected from the group consisting of glucuronic acid, mannuronic acid, and galacturonic acid.
5. The fermented product according to 3 above, wherein component Z2 is at least one compound selected from the group consisting of N-acetylneuraminic acid, glucuronic acid, mannuronic acid, and galacturonic acid.
6. The fermented product according to either of 3 and 5 above, further comprising component W, wherein component W is an isomer of component X, and component W is a different substance from component Z2.
7. The fermented product according to any one of 1-6 above, further comprising component V, wherein component V is a substance that is detected at RT27.0-35.0 when the fermented product is measured with HPLC-CAD, and component V is a different substance from component Z2.
8. The fermented product according to either of 4 and 5 above, wherein the ratio (content of component Z2 / content of component X) of the content of each compound of component Z2 relative to the content of component X in the fermented product is 0.02 or less on a mass basis.
9. The fermented product according to any one of 1-8 above, wherein component Z1 is at least one compound of glucose and lactose.
10. The fermented product according to 9 above, wherein the ratio (content of component Z1 / content of component X) of the content of each compound of component Z1 relative to the content of component X in the fermented product is 0.03 or less on a mass basis.
11. The fermented product according to 6 above, wherein the ratio (content of component W / content of component X) of the content of each compound of component W relative to the content of component X in the fermented product is 0.0035 or less on a mass basis.
12. The fermented product according to 7 above, wherein the ratio (content of component V / content of component X) of the total content of component V relative to the content of component X in the fermented product is 0.040 or less on a mass basis.
13. A method for producing a fermented product, the method comprising 1) to 4) below, in this order,
   1) treating solution A, which contains components X, Y, and Z, with a strongly acidic cation exchange resin to obtain solution B,
   2) treating solution B with a weakly basic anion exchange resin to obtain solution C,
   3) adjusting the pH of solution C to obtain solution D, and
   4) treating solution D with a weakly basic anion exchange resin to obtain solution E,

   wherein: component X is a sialic acid or a sialylated oligosaccharide;
   component Y is acetic acid;
   component Z is at least one of component Z1 and component Z2, and component Z is a component other than component X; and
   component Z1 is a neutral sugar and component Z2 is an acidic sugar;
   the ratio (total mass of component X in solution E / total mass of component X in solution A) of the total mass of component X in solution E relative to the total mass of component X in solution A is 0.5 or more; and
   the ratio (content of component Y / content of component X) of the content of component Y relative to the content of component X in solution E is 0.010 or less on a mass basis.
14. The method for producing a fermented product according to 13 above, further comprising 5) below,
   5) powderizing solution E,
   wherein: the fermented product contains components X, Y, and Z;
   the content ratio of component X per unit of solid matter in the fermented product is 80 mass% or more; and
   the ratio (content of component Y / content of component X) of the content of component Y relative to the content of component X in the fermented product is 0.010 or less on a mass basis.
15. The method for producing a fermented product according to either of 13 and 14 above, wherein the ratio (total mass of component Y in solution E / total mass of component Y in solution A) of the total mass of component Y in solution E relative to the total mass of component Y in solution A is 0.5 or more.
16. The method for producing a fermented product according to any one of 13-15 above, wherein component X is N-acetylneuraminic acid.
17. The method for producing a fermented product according to any one of 13-15 above, wherein component X is at least one compound selected from the group consisting of sialyllactose, sialyllacto-N-biose (SLNB), sialyllacto-N-acetylglucosamine (SLNac), sialyllacto-N-tetraose (LST), and disialyllacto-N-tetraose (DSLNT).
18. The method for producing a fermented product according to 16 above, wherein component Z2 is at least one compound selected from the group consisting of glucuronic acid, mannuronic acid, and galacturonic acid.
19. The method for producing a fermented product according to 17 above, wherein component Z2 is at least one compound selected from the group consisting of N-acetylneuraminic acid, glucuronic acid, mannuronic acid, and galacturonic acid.
20. The method for producing a fermented product according to either of 17 and 19 above, wherein the fermented product further comprises component W, component W is an isomer of component X, and component W is a different substance from component Z2.
21. The method for producing a fermented product according to any one of 13-20 above, wherein the fermented product further comprises component V, component V is a substance that is detected at RT27.0-35.0 when the fermented product is measured with HPLC-CAD, and component V is a different substance from component Z2.
22. The method for producing a fermented product according to either of 18 and 19 above, wherein the ratio (content of component Z2 / content of component X) of the content of each compound of component Z2 relative to the content of component X in the fermented product is 0.02 or less on a mass basis.
23. The method for producing a fermented product according to any one of 13-22 above, wherein component Z1 is at least one compound of glucose and lactose.
24. The method for producing a fermented product according to 23 above, wherein the ratio (content of component Z1 / content of component X) of the content of each compound of component Z1 relative to the content of component X in the fermented product is 0.03 or less on a mass basis.
25. The method for producing a fermented product according to 13 above, wherein 2) above comprises 2-1) to 2-3) below, in this order.
   2-1) passing solution B through a weakly basic anion exchange resin to remove at least a portion of neutral impurities that do not adsorb onto the weakly basic anion exchange resin,
   2-2) eluting at least a portion of the impurities remaining after 2-1) from the weakly basic anion exchange resin, and
   2-3) separately eluting acidic compounds containing component X and the impurities remaining after 2-2) from the weakly basic anion exchange resin
26. The method for producing a fermented product according to 13 above, wherein the liquid flow temperature of the weakly basic anion exchange resin in 2-2) above is 20°C or higher.
27. The method for producing a fermented product according to either of 25 and 26 above, wherein the liquid flow temperature of the weakly basic anion exchange resin in 2-3) above is 20°C or lower.
28. The method for producing a fermented product according to any one of 25-27 above, wherein the liquid flow temperature of the weakly basic anion exchange resin in 2-1) above is 15°C or lower.
29. The method for producing a fermented product according to either of 25 and 26 above, wherein: the fermented product further comprises component V, component V is a substance that is detected at RT27.0-35.0 when the fermented product is measured with HPLC-CAD, and component V is a different substance from component Z2; and
   the ratio (content of component V / content of component X) of the total content of component V relative to the content of component X in the fermented product is 0.040 or less on a mass basis.
30. The method for producing a fermented product according to either of 25 and 27 above, wherein: the fermented product further comprises component W, component W is an isomer of component X, and component W is a different substance from component Z2; and
   the ratio (content of component W / content of component X) of the content of each compound of component W relative to the content of component X in the fermented product is 0.0035 or less on a mass basis.
31. The method for producing a fermented product according to 25 above, wherein the liquid flow temperature of the weakly basic anion exchange resin in the step of 2-1) above is 5-15°C, the liquid flow temperature of the weakly basic anion exchange resin in the step of 2-2) above is 20-25°C, and the liquid flow temperature of the weakly basic anion exchange resin in the step of 2-3) above is 8-20°C.
32. The method for producing a fermented product according to 25 above, wherein the liquid flow temperature of the weakly basic anion exchange resin in 2-2) above is 20-25°C.
33. The method for producing a fermented product according to either of 25 and 26 above, wherein the liquid flow temperature of the weakly basic anion exchange resin in 2-3) above is 8-20°C.
34. The method for producing a fermented product according to any one of 25-27 above, wherein the liquid flow temperature of the weakly basic anion exchange resin in 2-1) above is 5-15°C.
35. The method for producing a fermented product according to 13 above, wherein the adjustment of the pH of solution C in 3) above is a method for passing solution C through a cation-exchange resin, and the liquid flow temperature of the cation exchange resin in 3) above is 20°C or lower.
36. The method for producing a fermented product according to either of 13 and 35 above, wherein the liquid flow temperature of the weakly basic anion exchange resin in 4) above is 20°C or lower.
37. The method for producing a fermented product according to either of 13 and 31 above, wherein the adjustment of the pH of solution C in 3) above is a method for passing solution C through a cation-exchange resin,
   the liquid flow temperature of the cation exchange resin in 3) above is 20°C or lower, and
   the liquid flow temperature of the weakly basic anion exchange resin in 4) above is 20°C or lower.

### EFFECT OF THE INVENTION

The fermented product produced by the method for producing a fermented product according to an embodiment of the present invention has a high content ratio of a sialic acid or sialylated oligosaccharide per unit of solid matter in the fermented product, and has a low acetic acid content. Furthermore, it is possible to produce a fermented product with less residual solvent than that of a crystallization process.

### BRIEF DESCRIPTION OF THE DRAWINGS

[Fig. 1] Fig. 1 is a graph showing the results of analyzing the composition of the eluate obtained from Comparative Example 1.
[Fig. 2] Fig. 2 is a graph showing the results of analyzing the composition of the eluate obtained from Example 1.
[Fig. 3] Fig. 3 is a graph showing the results of analyzing the composition of the eluate obtained from Comparative Example 2.
[Fig. 4] Fig. 4 is a graph showing the results of analyzing the composition of the eluate obtained from Example 2.

### MODE FOR CARRYING OUT THE INVENTION

The present invention will be described in detail below; however, these descriptions are intended to illustrate preferred embodiments and are not intended to limit the scope of the invention.
The symbol "-" in ranges of numerical values denotes a range that includes the numerical values immediately preceding and following it; for example, "0 mass%-100 mass%" means a range of 0 mass% or more and 100 mass% or less.

### [1. Fermented Product]

A fermented product according to an aspect of the present invention is a fermented product containing components X, Y, and Z,
wherein: component X is a sialic acid or a sialylated oligosaccharide;
the content ratio of component X per unit of solid matter in the fermented product is 80 mass% or more; and
component Y is acetic acid;
the ratio (content of component Y / content of component X) of the content of component Y relative to the content of component X in the fermented product is 0.010 or less on a mass basis;
component Z is at least one of component Z1 and component Z2, and component Z is a component other than component X; and
component Z1 is a neutral sugar and component Z2 is an acidic sugar.

Fermented products are mixtures of multiple compounds obtained through microbial fermentation. Compounds that may be contained in fermented products include, for example, a produced compound X, by-products of compound X, intermediates of compound X, degradation products of compound X or its intermediates, compound Y, compound Z, compound W, compound V, and culture medium components, etc.
The term "fermented product" is a concept that encompasses solution E, which is obtained through the first to fourth steps described below, powders, which are obtained through the first to fifth steps described below, and powders obtained by, in the fifth step, powderizing solutions obtained by concentrating, decolorizing, and/or filtering the solutions obtained in the first to fourth steps.

The solid content of the fermented product refers to the solid fraction remaining after volatile substances such as water have been removed from the fermented product, i.e., the residue after evaporation, and may include non-volatile components such as sialic acids, sialylated oligosaccharides, and other impurities. Specifically, the solid content refers, for example, to a powder obtained by freeze-drying or spray-drying solution E treated in the first to fourth steps described below, corrected for water content using the Karl Fischer titration method described below, or to the evaporated residue remaining after the fermented product is evaporated and dried by heating, and preferably refers to a value corrected for water content using the Karl Fischer titration method described below. When the fermented product is a solution, it refers to the solid content obtained by removing volatile substances such as water from the solution, and preferably refers to a value corrected for water content using the Karl Fischer titration method described below.

### (Component X: Sialic acid or Sialylated Oligosaccharide)

A fermented product according to an aspect of the present invention contains a sialic acid or sialylated oligosaccharide as component X.

Sialic acid is a general term for substances in which the amino group or hydroxyl group of neuraminic acid has been substituted. Examples of sialic acids include N-acetylneuraminic acid, N-glycolylneuraminic acid, glucuronic acid, N-acetylgalactosamine-6-sulfate, and mannose-6-phosphate, etc.; and N-acetylneuraminic acid is preferred.

Sialylated oligosaccharides are acidic oligosaccharides formed by the binding of sialic acid and an oligosaccharide, preferably sialic acid and lactose, or sialic acid and a neutral sugar. Neutral sugars include glucose, galactose, lactose, and sucrose, with lactose being preferred. Examples of sialylated oligosaccharides include sialyllactose, sialyllacto-N-biose (SLNB), sialyllacto-N-acetylglucosamine (SLNac), sialyllacto-N-tetraose (LST), disialyllacto-N-tetraose (DSLNT), 3-fucosyl-sialyl-lactose (F-SL), and fucosyl-LSTb (F-LSTb), etc., and sialyl-lactose is preferred.

Sialyllactose is an acidic oligosaccharide formed by the binding of sialic acid to lactose. Examples of sialyllactose include 6'-sialyllactose [O-(N-acetyl-α-neuraminosyl)-(2→6)-O-β-D-galactopyranosyl-(1→4)-D-glucose] (hereinafter also referred to as "6SL") and 3'-sialyllactose [O-(N-acetyl-α-neuraminosyl)-(2→3)-O-β-D-galactopyranosyl-(1→4)-D-glucose] (hereinafter also referred to as "3SL").

Sialyllacto-N-biose is an acidic oligosaccharide formed by the binding of sialic acid to lacto-N-biose. Examples of sialyllacto-N-biose include 6'-sialyl-lacto-N-biose (hereinafter also referred to as "6SLNB") and 3'-sialyl-lacto-N-biose (hereinafter also referred to as "3SLNB").

Sialyllacto-N-acetylglucosamine is an acidic oligosaccharide formed by the binding of sialic acid to lacto-N-acetylglucosamine. Examples of sialyl-N-acetylglucosamine include 6'-sialyl-N-acetylglucosamine (hereinafter also referred to as "6SLNAc") and 3'-sialyl-N-acetylglucosamine (hereinafter also referred to as "3SLNAc").

Sialyllacto-N-tetraose is an acidic oligosaccharide formed by the binding of sialic acid to lacto-N-tetraose or lacto-N-neotetraose. Examples of sialyllacto-N-tetraose include LST-a, LST-b, and LST-c.

Disialyllacto-N-tetraose is an acidic oligosaccharide formed by the binding of two molecules of sialic acid to lacto-N-tetraose.

In one aspect of the present invention, the content of component X per unit of solid matter in the fermented product is 80% by mass or more, and, in order of preference, is 82 mass% or more, 84 mass% or more, 85 mass% or more, 87 mass% or more, 88 mass% or more, 89 mass% or more, and 90 mass% or more. Note that in this specification, the term "content of component X" is synonymous with the "purity of component X".

When component X is a sialic acid, the content ratio of component X per unit of solid matter in the fermented product in an aspect of the present invention is 80 mass% or more, preferably 84 mass% or more, more preferably 87 mass% or more, still more preferably 88 mass% or more, still more preferably 89 mass% or more, and most preferably 90 mass% or more. When component X is a sialylated oligosaccharide, the content ratio of component X per unit of solid matter in the fermented product in an aspect of the present invention is 80 mass% or more, preferably 84 mass% or more, and more preferably 87 mass% or more. In particular, when component X is at least one of 3SL and 6SL, the content ratio of component X per solid content of the fermented product in an aspect of the present invention is preferably 87 mass% or more.

The sialic acids and sialylated oligosaccharides of component X may be contained in the fermented product as sialates and sialylated oligosaccharide salts, respectively. In this specification, when calculating the content ratio and content ratio of component X, a value obtained by converting the amount of sialate to the equivalent number of moles of sialic acid, or a value obtained by converting the amount of sialylated oligosaccharide salt to the equivalent number of moles of sialylated oligosaccharide (hereinafter also referred to as the free form), is used.

The content ratio of component X per unit of solid matter in the fermented product refers to the proportion (purity) of component X in the solid matter and can be calculated from the moisture content measured by the Karl Fischer titration method, and the content of component X measured by a High Performance Liquid Chromatography-Charged Aerosol Detector (hereinafter also referred to as an "HPLC-CAD"). More specifically, examples include the methods described in the analysis examples and examples below. When the fermented product is a solution, the content ratio can be determined by evaporating to dryness. Note that the content of component X in the fermented product refers to the amount (concentration) of component X in a solution.

Furthermore, when the fermented product is a solution, the content ratio of component X can be calculated by subtracting the content of impurities in the fermented product from the 100% mass content ratio of component X in the fermented product. More specifically, examples include the methods described in the analysis examples and examples below.

The content of component X in the fermented product refers to the amount (concentration) of component X in a solution such as solution E described below, and this can be measured and calculated using HPLC-CAD. More specifically, examples include the methods described in the analysis examples and examples below.

### (Component Y: Acetic Acid)

A fermented product according to an aspect of the present invention further includes acetic acid as component Y.

Acetic acid is an organic acid produced during microbial fermentation and, like component X, is an acidic substance. Therefore, when a process for increasing the content ratio of component X is performed during a microbial fermentation process, the acetic acid content may increase as it exhibits behavior similar to that of component X. Thus, by performing a process for separating component X and component Y, the acetic acid content in the fermented product can be reduced, and the content ratio of component X can be increased. Such a treatment makes it possible to provide a fermented product in which impurities have been sufficiently reduced.

In particular, in one aspect of the present invention, the ratio (content of component Y / content of component X) of the content of component Y relative to the content of component X in the fermented product is 0.010 or less on a mass basis, preferably 0.008 or less, more preferably 0.006 or less, and most preferably 0.004 or less.

The sialic acids and sialylated oligosaccharides of component X may be contained in the fermented product as sialates and sialylated oligosaccharide salts, respectively. Furthermore, component Y, acetic acid, may be contained in the fermented product as an acetate. In this specification, when calculating the (content of component Y / content of component X), a value obtained by converting the amount of sialate to the equivalent number of moles of sialic acid, or a value obtained by converting the amount of sialylated oligosaccharide salt to the equivalent number of moles of sialylated oligosaccharide, and a value obtained by converting the amount of acetate into the equivalent number of moles of acetic acid, are used.

Examples of methods for calculating the ratio (content of component Y / content of component X) of the content of component Y relative to the content of component X in the fermented product include the methods described in the analysis examples and examples below.

### (Component Z: Neutral Sugars and Acidic Sugars)

A fermented product according to an aspect of the present invention further contains at least one of component Z1 and component Z2 as component Z. Component Z is a different compound from component X. Component Z1 is a neutral sugar and component Z2 is an acidic sugar.

### (Component Z1: Neutral Sugar)

Examples of neutral sugars contained as component Z1 include, for example, glucose, galactose, lactose, and sucrose, which are used in fermentative production, as well as fructose, N-acetylmannosamine, N-acetylglucosamine, and the like, which are produced by microorganisms used in fermentative production. In particular, glucose and sucrose, which are added during the cultivation of microorganisms used for fermentation, as well as lactose, which is added to the culture broth during the production of sialylated oligosaccharides, may have high concentrations at the end of fermentation. In particular, removing glucose and lactose from the fermented product can increase the content ratio of component X in the fermented product.

In a fermented product according to one aspect of the present invention, it is preferable that the content of impurities such as neutral sugars is low, and the ratio (content of component Z1 / content of component X) of the content of each compound of component Z1 relative to the content of component X in the fermented product is preferably 0.03 or less on a mass basis, more preferably 0.01 or less, still more preferably 0.001 or less, and most preferably 0.0005 or less.

"The ratio (content of component Z1 / content of component X) of the content of each compound of component Z1 relative to the content of component X in the fermented product is preferably 0.03 or less on a mass basis" means, for example, that when component Z1 consists solely of glucose, the ratio (content of glucose / content of component X) of the content of glucose relative to the content of component X in the fermented product is preferably 0.03 or less. Further, when component Z1 consists solely of lactose, the ratio (content of lactose / content of component X) of the content of lactose relative to the content of component X in the fermented product is preferably 0.03 or less. In addition, when component Z1 consists of glucose and lactose, the ratio (content of glucose / content of component X) of the content of glucose relative to the content of component X in the fermented product is preferably 0.03 or less, and the ratio (content of lactose / content of component X) of the content of lactose relative to the content of component X in the fermented product is preferably 0.03 or less.

Examples of methods for calculating the ratio (content of component Z1 / content of component X) of the content of each compound of component Z1 relative to the content of component X in the fermented product include the methods described in the analysis examples and examples below.

### (Component Z2: Acidic Sugar)

Examples of acidic sugars contained as component Z2 include N-acetylneuraminic acid, N-glycolylneuraminic acid, glucuronic acid, N-acetylgalactosamine-6-sulfate, and mannose-6-phosphate, etc., produced by microorganisms used in fermentative production, as well as N-acetylneuraminic acid produced generated during the production of sialylated oligosaccharides, and sialylated oligosaccharides other than component X to which N-acetylneuraminic acid is bound. Examples of other acidic sugars contained as component Z2 include mannuronic acid, galacturonic acid, and L-isuronic acid. Similar to component Y, these acidic sugars possess physical properties similar to those of component X, particularly in terms of acidity; therefore, they are readily adsorbed and eluted from basic anion exchange resins together with component X. Consequently, during the production of fermented products containing component X, component Z2 tends to remain in the fermented product as an impurity; however, through appropriate processing, it is possible to increase the content ratio of component X and reduce the amount of acidic sugars. In particular, it is preferable that the content of glucuronic acid, mannuronic acid, and galacturonic acid is low; furthermore, when component X is a sialylated oligosaccharide, it is preferable that the content of N-acetylneuraminic acid is low.

As described above, component Z, such as component Z2, is different from component X. Therefore, when the fermented product according to an aspect of the present invention contains N-acetylneuraminic acid as component X, the N-acetylneuraminic acid is not component Z, such as component Z2. On the other hand, for example, when the fermented product according to an aspect of the present invention contains a sialylated oligosaccharide such as sialyllactose and N-acetylneuraminic acid, if the sialylated oligosaccharide such as sialyllactose is component X, then N-acetylneuraminic acid is component Z2.

In a fermented product according to one aspect of the present invention, it is preferable that the content of impurities such as acidic sugars is low, and when component Z2 is at least one substance selected from the group consisting of glucuronic acid, mannuronic acid, galacturonic acid, and N-acetylneuraminic acid, the ratio (content of component Z2 / content of component X) of the content of each compound of component Z2 relative to the content of component X in the fermented product is preferably 0.02 or less on a mass basis, and more preferably 0.01 or less.

"The ratio (content of component Z2 / content of component X) of the content of each compound of component Z2 relative to the content of component X in the fermented product is preferably 0.02 or less on a mass basis" means, for example, that when component Z2 consists solely of N-acetylneuraminic acid, the ratio (content of N-acetylneuraminic acid / content of component X) of the content of N-acetylneuraminic acid relative to the content of component X in the fermented product is preferably 0.02 or less. When component Z2 consists solely of glucuronic acid, the ratio (content of glucuronic acid / content of component X) of the content of glucuronic acid relative to the content of component X in the fermented product is preferably 0.02 or less. In addition, when component Z2 consists of N-acetylneuraminic acid and glucuronic acid, the ratio (content of N-acetylneuraminic acid / content of component X) of the content of N-acetylneuraminic acid relative to the content of component X in the fermented product is preferably 0.02 or less, and the ratio (content of glucuronic acid / content of component X) of the content of glucuronic acid relative to the content of component X in the fermented product is preferably 0.02 or less.

Examples of methods for calculating the ratio (content of component Z2 / content of component X) of the content of each compound of component Z2 relative to the content of component X in the fermented product include the methods described in the analysis examples and examples below.

### (Component W: Isomers of Component X)

Examples of component X contained as component W include, when component X is a sialylated oligosaccharide, compounds in which the aldose monosaccharide unit at the reducing end of the sialylated oligosaccharide produced during the fermentation and purification processes has been converted to a ketose. Examples of compounds in which the aldose monosaccharide unit at the reducing end of the sialylated oligosaccharide has been converted to a ketose include 3'-sialyllactulose and 6'-sialyllactulose. Component W is a different substance from component Z2.

The formation of isomers of component X is promoted under high-temperature storage conditions exceeding 20°C. Therefore, when producing a fermented product containing component X, it is possible to enhance the content ratio of component X by controlling the production of isomers of component X as a result of setting the liquid flow temperature of the weakly basic anion exchange resin to be 20°C or lower in the step described below of 2-3) separately eluting acidic compounds containing component X and the impurities remaining after 2-2) from the weakly basic anion exchange resin.

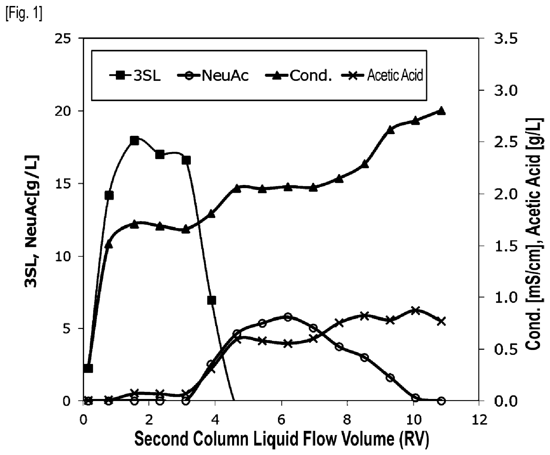

Furthermore, these isomers of component X may be produced under storage conditions at pH 4.8 or higher. For example, fig. 1 of International Publication 2020/148693 shows the amount of 6'-sialyllactulose formed in freeze-dried 6SL samples having various pH values during an accelerated thermal stability test. Since 6SL is isomerized during storage and converted to 6'-sialyllactulose, sialylated oligosaccharides exhibiting improved chemical stability and/or physical properties can be obtained by reducing the initial amount of sialylated oligosaccharide isomers during treatment with ion exchange resins.

In a fermented product according to one aspect of the present invention, it is preferable that the content of impurities such as component W is low, examples of the ratio (content of component W / content of component X) of the content of component W relative to the content of component X in the fermented product include 0.0080 or less, 0.0070 or less, 0.0060 or less, 0.0050 or less, 0.0040 or less, 0.0038 or less, 0.0035 or less, 0.0030 or less, 0.0025 or less, 0.0020 or less, and 0.0010 or less on a mass basis, and the value is preferably 0.0038 or less, more preferably 0.0035 or less, still more preferably 0.0030 or less, and most preferably 0.0025 or less. In other embodiments, the range is 0.00010-0.0080, 0.00010-0.0040, 0.000010-0.0035, 0.000010-0.0030, or 0.000010-0.0025.

Examples of methods for calculating the ratio (content of component W / content of component X) of the content of each compound of component W relative to the content of component X in the fermented product include the methods described in the analysis examples and examples below.

### (Component V: Substances Detected at RT27.0-35.0 When Fermented Product Is Measured with HPLC-CAD)

Component V is a general term for substances detected at RT 27.0-35.0 when the fermented product is measured with HPLC-CAD, as described in the analysis examples below, and contains one or more components. Component V is a different substance from component Z2. Specifically, examples include acidic sugars in which one or more sialic acids are bound to oligosaccharides composed of 2 to 10 monosaccharides. Examples of component V when component X is 3'-sialyllactose or 6'-sialyllactose include sialyllacto-N-tetraose (LST) and disialyllacto-N-tetraose (DSLNT), etc.

Similar to component Y, component V possesses physical properties similar to those of component X, particularly in terms of acidity; therefore, it is readily adsorbed and eluted from basic anion exchange resins together with component X.

Consequently, during the production of fermented products containing component X, component V tends to remain in the fermented product as an impurity; however, through appropriate processing, it is possible to improve the removal rate of component V and increase the content ratio of component X in the fermented product.

A method for improving the removal rate of component V is a method for setting the liquid flow temperature of the weakly basic anion exchange resin to 20°C or higher in the step described below of 2-2) eluting at least a portion of the impurities remaining after 2-1) from the weakly basic anion exchange resin. This allows for the separation of component V which has a weaker adsorption capacity to the weakly basic anion exchange resin than component X.

Component V is a substance that can be produced by microorganisms used in fermentative production. As the composition of solution A prior to the first step, the ratio [(content (g/L) of component V per 1 L of solution A × liquid volume (L) of solution A) / volume (L) of weakly basic ion exchange resin] of the total mass of component V relative to the volume of the weakly basic ion exchange resin used in the second step is typically 5 g/L or more, preferably 10 g/L or more, more preferably 15 g/L or more, still more preferably 17 g/L or more, even more preferably 20 g/L or more, and most preferably 25 g/L or more. When within the above ranges, the content ratio of component X can be increased by using a method for improving the removal rate of component V.

In a fermented product according to one aspect of the present invention, it is preferable that the content of impurities such as component V is low, and the ratio (content of component V / content of component X) of the total content of component V relative to the content of component X in the fermented product is preferably 0.040 or less on a mass basis, more preferably 0.038 or less, still more preferably 0.036 or less, even more preferably 0.034 or less, and most preferably 0.032 or less. In other embodiments, the range is 0.0010-0.040, 0.0010-0.038, 0.0010-0.036, 0.0010-0.034, or 0.0010-0.032.

Examples of methods for calculating the ratio (content of component V / content of component X) of the total content of component V relative to the content of component X in the fermented product include the methods described in the analysis examples and examples below.

When the removal rate of component V is improved, the content ratio of component X per unit of solid matter in the fermented product according to an aspect of the present invention is preferably 82 mass% or more, and, in order of preference, is 84 mass% or more, 85 mass% or more, 87 mass% or more, 88 mass% or more, 89 mass% or more, and 90 mass% or more.

### (Other Components)

The fermented product according to an aspect of the present invention may contain inorganic ions or inorganic salts, etc., in addition to the components described above. Many inorganic ions originate from components present during fermentative production, but most are removed during the process of increasing the content of component X. Therefore, apart from inorganic ions that act as cations serving as counterions to component X contained in the fermented product of one aspect of the present invention, only very small amounts, for example 10 mg/kg or less, of inorganic ions may be contained. In particular, since component X is an acidic substance, inorganic ions are selected as counterions for these acidic substances, for example as monovalent cations, and inorganic salts may be contained as monovalent cation salts in the fermented product according to one aspect of the present invention. Examples of monovalent cations include hydrogen ions, sodium ions, potassium ions, ammonium ions, and lithium ions, etc., but from the perspective of use in foods, and the like, sodium ions or potassium ions are preferred. Other inorganic ions include inorganic anions such as Cl⁻, PO₄³⁻, and SO₄²⁻.

### (Other Characteristics of Fermented Product)

It is preferable that the method for producing a fermented product according to an aspect of the present invention does not include a crystallization process.

For example, in the case of a method for producing component X by chemical synthesis and a method for purifying component X by crystallization, organic solvents such as ethanol, methanol, and toluene remain in a final product. On the other hand, a method for producing a fermented product according to an aspect of the present invention described below does not require the use of these organic solvents. Therefore, it is considered that a fermented product obtained by the production method of an aspect of the present invention is safer for the human body.

It is preferable that the fermented product according to an aspect of the present invention has a reduced content of compounds designated as "Class 3 solvents (solvents which should be limited by GMP or other quality-based requirements)" in the aforementioned "Guideline for Residual Solvents" of the International Council for Harmonisation of Technical Requirements for Pharmaceuticals for Human Use (ICH), in comparison with the content of these compounds in fermented products obtained by conventional techniques. In a fermented product of one aspect of the present invention, the ratio (content of class 3 solvents / content of target product) of the content of compounds designated as "Class 3 solvents (solvents which should be limited by GMP or other quality-based requirements)" in the fermented product is 0.010 or less on a mass basis, preferably 0.008 or less, and more preferably 0.006 or less. Acetic acid is an example of a class 3 solvent, but, as described above, it is preferable that the fermented product obtained by the production method of an aspect of the present invention has a low acetic acid content, and is safer for the human body in comparison with conventional fermented products.

When the fermented product according to an aspect of the present invention is a solution, the pH is preferably 1 to 7, more preferably 3 to 7, and most preferably 3 to 5. When the fermented product according to an aspect of the present invention is a powder, it is preferable that the pH is 3 to 5 when 1 g of the powder is dissolved in 0.01 L of water, and then 0.005 L of the solution is dispensed, and diluted twofold with an equal volume of 0.005 L of water.

### [2. Production Method of Solution, and Production Method of Fermented Product]

The term "1. Fermented Product" is a concept that encompasses solution E, which is obtained through the first to fourth steps described below, powders, which are obtained through the first to fifth steps described below, and powders obtained by, in the fifth step, powderizing solutions obtained by concentrating, decolorizing, and/or filtering the solutions obtained in the first to fourth steps.

A method for producing a fermented product according to an aspect of the present invention method contains 1) to 4) below, in this order,
1) treating solution A, which contains components X, Y, and Z, with a strongly acidic cation exchange resin to obtain solution B,
2) treating solution B with a weakly basic anion exchange resin to obtain solution C,
3) adjusting the pH of solution C to obtain solution D, and
4) treating solution D with a weakly basic anion exchange resin to obtain solution E.

In this instance: component X is a sialic acid or a sialylated oligosaccharide,
component Y is acetic acid;
component Z is at least one of component Z1 and component Z2, and component Z is a component other than component X;
component Z1 is a neutral sugar and component Z2 is an acidic sugar;
the ratio (total mass of component X in solution E / total mass of component X in solution A) of the total mass of component X in solution E relative to the total mass of component X in solution A is 0.5 or more; and
the ratio (content of component Y / content of component X) of the content of component Y relative to the content of component X in solution E is 0.010 or less on a mass basis.

A method for producing a fermented product according to an aspect of the present invention may further contain 5) below.
5) powderizing solution E
A fermented product obtained by this production method contains components X, Y, and Z,
the content ratio of component X per unit of solid matter in the fermented product is 80 mass% or more, and
the ratio (content of component Y / content of component X) of the content of component Y relative to the content of component X in the fermented product is 0.010 or less on a mass basis.

The above production method allows for a reduction in the content of component Y, and since the recovery rate of component X, that is, (total mass of component X in solution E / total mass of component X in solution A), is 0.5 or higher, the method is sufficiently industrially applicable that it is fully applicable, for example, to industrial production, for example, the production volume of the fermented product is on a kilogram scale. A high recovery rate of component X is preferable in order to achieve efficient production of component X, and the ratio (total mass of component X in solution E / total mass of component X in solution A) of the total mass of component X in solution E relative to the total mass of component X in solution A is preferably 0.5 or more, more preferably 0.6 or more, and still more preferably 0.7 or more.

The ratio (total mass of component X in solution E / total mass of component X in solution A) of the total mass of component X in solution E relative to the total mass of component X in solution A can be calculated by analysis using HPLC-CAD. More specifically, it can be calculated using the methods described in the analysis examples and examples described below.

In addition, the ratio (total mass of component Y in solution E / total mass of component Y in solution A) of the total mass of component Y in solution E relative to the total mass of component Y in solution A is preferably 0.5 or less, more preferably 0.4 or less, and most preferably 0.2 or less. When the ratio (total mass of component Y in solution E / total mass of component Y in solution A) of the total mass of component Y in solution E relative to the total mass of component Y in solution A falls within the above-mentioned ranges, it is considered that component Y has been efficiently removed from the fermented product, such as solution E.

The ratio (total mass of component Y in solution E / total mass of component Y in solution A) of the total mass of component Y in solution E relative to the total mass of component Y in solution A can be calculated using HPLC-UV. More specifically, it can be calculated using the methods described in the analysis examples and examples described below.

A method for producing a fermented product according to an aspect of the present invention need not include at least one of a chemical synthesis step of component X and a crystallization step of component X. As a result of not including a chemical synthesis or crystallization step, the possibility of the final fermented product containing organic solvents can be reduced.

In this specification, step 1) above is referred to as a first step, step 2) above as a second step, step 3) above as a third step, step 4) above as a fourth step, and step 5) above as a fifth step.

A fermented product according to an aspect of the present invention can be obtained by purifying, or similar, solution A, which contains components X, Y, and Z, using a method including the following steps 1) to 5). This solution A can be prepared by methods such as culturing microbes capable of producing component X.

As microbes capable of producing component X, microbes having the ability to produce a sialic acid or sialylated oligosaccharide such as sialyllactose are preferred.

The microbes having the ability to produce a sialic acid or sialylated oligosaccharide are not particularly limited as long as they can produce a sialic acid or sialylated oligosaccharide; and may be microbes that inherently have the ability to produce a sialic acid or sialylated oligosaccharide, or may be microbes that do not have the ability to produce a sialic acid or sialylated oligosaccharide, but into which genes necessary for the production of a sialic acid or sialylated oligosaccharide have been introduced. Examples of microbes having the ability to produce a sialic acid or sialylated oligosaccharide include, for example, recombinant microbes into which genes necessary for the production of a sialic acid or sialylated oligosaccharide have been introduced.

Examples of such microbes include Escherichia coli, Lactobacillus lactis, Corynebacterium glutamicum, Bacillus subtilis, Pseudomonas putida, and other bacteria, and Saccharomyces cerevisiae, Schizosaccharomyces pombe, Pichia pastoris, and other yeast, and among these, Escherichia coli is more preferable.

Examples of the Escherichia coli include Escherichia coli strain W3110 (ATCC 27325), strain MG1655 (ATCC 47076), strain BL21(DE3), and Escherichia coli W strain (ATCC 9637), and derivatives thereof.

Examples of genes necessary for the production of a sialic acid or sialylated oligosaccharide include, for example, genes encoding glucosamine-6-phosphate synthase, genes encoding glucosamine-6-phosphate N-acetyltransferase, genes encoding N-acetylglucosamine 2-epimerase, genes encoding UDP-N-acetylglucosamine 2-epimerase, genes encoding N-acetylneuraminic acid synthase, and genes encoding CMP-N-acetylneuraminic acid synthase. Examples of genes whose expression is preferably enhanced in microbes having the ability to produce a sialic acid or sialylated oligosaccharide include, for example, genes encoding glucosamine-6-phosphate synthase, genes encoding phosphoenolpyruvate synthase, and genes encoding pyruvate carboxylase. Examples of genes whose expression is preferably reduced or absent in microbes having the ability to produce a sialic acid or sialylated oligosaccharide include, for example, genes encoding β-galactosidase, genes encoding the nanRATEK enzyme family, genes encoding glucosamine-6-phosphate deaminase, and genes encoding PTS transporters such as manXYZ and nagE.

Methods for producing microbes having the ability to produce a sialic acid or sialylated oligosaccharide include those described in International Publication No. 2019/228993, Japanese Unexamined Patent Application Publication (Translation of PCT Application) No. 2021-505170, and Japanese Unexamined Patent Application Publication (Translation of PCT Application) No. 2020-531039, among others.

### (Preliminary Stage Prior to First Step)

Solution A, which contains components X, Y, and Z, is used in the first step. Solution A may further contain at least one component selected from the group consisting of component Z1, component Z2, component W, and component V. Solution A preferably contains a culture broth produced by microbes that produce component X. Prior to performing the first step, a step for separating solid matter containing microbes from culture broth and recovering the culture supernatant containing component X may be performed. Examples of methods for separating solid matter containing microbes from culture broth include centrifugation, filtration, and cross-flow filtration, etc. In this specification, the term "culture broth" refers to a mixture containing microbes, a culture medium, components X, Y, and Z, and the like. The term "culture supernatant" refers to a liquid obtained after separating the solid matter containing microbes from the culture broth.

The composition of solution A prior to the first step includes, for example: sialyllactose as component X, with the content of component X being 0.1 g/L to 100 g/L; N-acetylneuraminic acid as component Z2, with the ratio (content of component Z2) / (content of component X) of the content of component 2 relative to the content of component X being 0.03 to 0.5 on a mass basis; and acetic acid as component Y, with the ratio (content of component Y / content of component X) of the content of component Y relative to the content of component X being 0.010 to 0.60 on a mass basis.

### (First Step)

In the first step, solution A, which contains components X, Y, and Z, is treated with a strongly acidic cation exchange resin to obtain solution B.

Specifically, in the first step, solution A, for example, a culture supernatant obtained by microbial fermentation, is treated with a strongly acidic cation exchange resin to adsorb impurities of microbial origin and positively charged contaminants onto the resin, thereby removing them from solution A. Examples of positively charged contaminants include Na⁺, K⁺, and NH₄⁺, etc., while examples of microbial impurities include nucleic acids, amino acids, and proteins, etc.

The strongly acidic cation exchange resin is not particularly limited, and e.g., examples include strongly acidic cation exchange resins having sulfonic acid groups as exchange groups of the strongly acidic cation exchange resin. Furthermore, examples of matrix materials for the strongly acidic cation exchange resin include porous types, macroporous types, gel types, styrene types, and acrylic types, etc.

Specific examples of strongly acidic cation exchange resins include, e.g., the DIAION SK series (SK1B, SK1BH, SK1BL, SK1BLH, SKL10, SKT10L, SKT110L, SK110L, SK112L, SKT20L, SK104, SK110, SK112, SK116, etc.) manufactured by MITSUBISHI CHEMICAL CORPORATION, the DIAION PK series (e.g., PK208, PK212, PK216, PK220, PK228, PK208LH, PK212L, PK212LH, PK216L, PK216H, PK216LH, PK220L, PK228L, PK228LH, etc.) manufactured by MITSUBISHI CHEMICAL CORPORATION, DIAION RCP series (e.g., RCP145H, RCP160M, etc.) manufactured by MITSUBISHI CHEMICAL CORPORATION, Diaion HPK25 manufactured by MITSUBISHI CHEMICAL CORPORATION, the Diaion UBK series (e.g., UBK16, UBK14, UBK12, UBK10, UBK08, UBK04, UBK02, UBK10H, UBK10HUP, UBK08A, UBK08H, UBK08HUP, UBKN1U, UBKN1UMB, UBK522M, UBK530, UBK530J, UBK530K, UBK535, UBK535J, UBK535K, UBK535L, UBK550, UBK555, etc.) manufactured by MITSUBISHI CHEMICAL CORPORATION, the RELITE JC series (e.g., JC600, JC603, etc.) manufactured by MITSUBISHI CHEMICAL CORPORATION, S1668 manufactured by LANXESS PTE. LTD., the LEWATIT MONOPLUS series (e.g., S108, S108H, SP112, etc.) manufactured by LANXESS PTE. LTD., DOWEX 88, DOWEX 88MB, DOWEX Monosphere 88, and TG-GEL (also known as XUS40232-01) manufactured by THE DOW CHEMICAL COMPANY, AMBERLITE (e.g., FPC16 UPS Na, FPC88 MB Na, FPC240H, CR3220 Ca, CR1310 Ca, Na, CR1360 Na, CR99K/350, HPR1100 Na, etc.) manufactured by DUPONT DE NEMOURS, INC., and C100, C100E, C120E, C100×10, C100×16MBH, C145S, C150, C160, SGC650, and the PUROLITE SST series (e.g., SSTC60, SSTC60H, SSTC80C, etc.) manufactured by PUROLITE.

As the strongly acidic cation exchange resin, it is preferable that the exchange groups in the strongly acidic cation exchange resin have sulfonic acid groups, and a gel type is more preferable as the matrix of the strongly acidic cation exchange resin. Specifically, examples include UBK04 and TG-Gel (also known as XUS40232-01). Furthermore, the ionic form of the sulfonic acid group as the exchange group in the strongly acidic cation exchange resin is not particularly limited, and examples include H⁺, Na⁺, K⁺, and NH₄⁺, and H⁺ is preferred.

The pH of solution A, for example, a culture supernatant obtained by microbial fermentation, for treatment with the strongly acidic cation exchange resin is preferably 1 to 7, and more preferably 3 to 5. The concentration of component X in solution A is preferably 0.1 g/L to 100 g/L, more preferably 1 g/L to 70 g/L, and most preferably 10 g/L to 50 g/L. If solution A, for example, a culture broth obtained by microbial fermentation, does not have the above-mentioned preferred pH or concentration of component X, solution A may be pretreated. Examples of pretreatment include pH adjustment and ultrafiltration of solution A.

In the first step, in addition to solution A, examples of solutions that can be used for liquid flow when treating solution A with a strongly acidic cation exchange resin include deionized water, and an eluent. Deionized water is used to elute substances that do not adsorb to the strongly acidic cation exchange resin, thereby improving the recovery rate of component X.

In this step, solution A and deionized water are preferred as solutions that can be used for elution when treating solution A with a strongly acidic cation exchange resin.

In this specification, the flow rate when passing a solution through an ion exchange resin is defined by space velocity (the volume ratio of solution passing through the column per hour when the resin volume of the ion exchange resin is 1; hereinafter referred to as "SV").

As liquid flow conditions for treating solution A, for example, a culture broth obtained by microbial fermentation, with a strongly acidic cation exchange resin, the flow rate is preferably SV 0.1 to 5, more preferably SV 0.2 to 4, and most preferably SV 0.5 to 2. Under these liquid flow conditions, the temperature is preferably 3 to 30°C, more preferably 5 to 25°C, and most preferably 8 to 20°C.

Furthermore, in this specification, the liquid flow temperature of the ion exchange resin refers to the temperature when at least one temperature among the temperature of a solution introduced from an inlet of a column packed with the ion exchange resin, the temperature of a solution discharged from an outlet of the column, or the temperature of an apparatus used in a column packed with the ion exchange resin, is equilibrated to either room temperature or the temperature of a chromatography chamber.

An eluate that has passed through the strongly acidic cation exchange resin, that is, solution B, is recovered and used in the second step described below.

### (Second Step)

In the second step, solution B is treated with a weakly basic anion exchange resin to obtain solution C.

Specifically, in the second step, component X and the contaminants described below are separated by treating solution B (of the strongly acidic cation exchange resin in the first step), which was obtained by the first step, with a weakly basic anion exchange resin. The second step involves passing solution B, which was obtained in the first step, through a weakly basic anion exchange resin, and more specifically, involves 2-1) passing solution B through a weakly basic anion exchange resin to remove at least a portion of neutral impurities that do not adsorb onto the weakly basic anion exchange resin, 2-2) eluting at least a portion of the impurities remaining after 2-1) from the weakly basic anion exchange resin, and 2-3) separately eluting acidic compounds containing component X and the impurities remaining after 2-2) from the weakly basic anion exchange resin.

Examples of neutral impurities include, e.g., the neutral sugars of component Z1.

Examples of acidic compounds containing component X include, in addition to component X, negatively charged impurities such as anions including SO₄²⁻, Cl⁻, and PO₄³⁻, as well as component Y, organic acids other than component Y such as formic acid and fatty acids, nucleic acids, amino acids, proteins, and phospholipids.

The weakly basic anion exchange resin is not particularly limited, and e.g., examples include ion exchange resins having primary to tertiary amino groups or polyamino groups as exchange groups of the weakly basic anion exchange resin. Furthermore, examples of matrix materials for the weakly basic anion exchange resin include porous types, macroporous types, gel types, styrene types, and acrylic types, etc.

Specific examples of weakly basic anion exchange resins include, for example, the DOWEX (registered trademark) MONOSPHERE series (e.g., MONOSPHERE 77) manufactured by THE DOW CHEMICAL COMPANY, PUROLITE (registered trademark) A100, A103S, A110, A111S, A133S, A830W, A845, and A847 manufactured by PUROLITE, HPR4780, IRA67, IRA96SB, and IRA98 manufactured by ORGANO CORPORATION, AMBERLITE (e.g., FPA77UP, XE583GF, FPA53, HPR4780 CI, IRA67, IRA96SB, IRA98, FPA95, FPA96, FPA77UPS, XE583GF, and FPA53, etc.) manufactured by DUPONT DE NEMOURS, INC., the Diaion (registered trademark) WA Series (e.g., WA10, WA20, WA21J, WA30, WA30C, WA30LL, and WA55, etc.) manufactured by MITSUBISHI CHEMICAL CORPORATION, SEPABEADS FPDA13 manufactured by MITSUBISHI CHEMICAL CORPORATION, the RELITE JA series (e.g., JA300, JA310, JA450, and JA830, etc.) manufactured by MITSUBISHI CHEMICAL CORPORATION, LEWATIT MP62WS manufactured by LANXESS PTE. LTD., and LEWATIT MONOPLUS MP64 manufactured by LANXESS PTE. LTD., etc.

As the weakly basic anion exchange resin, ion exchange resins in which the exchange groups of the weakly basic anion exchange resin have tertiary amino groups are more preferable. Specifically, examples of ion exchange resins in which the exchange groups of the weakly basic anion exchange resin have tertiary amino groups include PUROLITE (registered trademark) A845/1967, A830W, etc. manufactured by PUROLITE.

Furthermore, the ionic form of the tertiary amino groups serving as the exchange groups in the weakly basic anion exchange resin is not particularly limited, and e.g., examples include hydroxide ions (OH⁻ type), chloride ions (Cl⁻ type), and organic acids having two or more carbon atoms, and hydroxide ions (OH⁻ type) are preferred.

Specifically, the organic acids having two or more carbon atoms are preferably carboxylic acids having two or more carbon atoms. Examples of organic acids having two or more carbon atoms include acetic acid, propionic acid, butyric acid, valeric acid, lactic acid, glycolic acid, pyruvic acid, gluconic acid, tartaric acid, oxalic acid, malonic acid, succinic acid, glutaric acid, adipic acid, malic acid, fumaric acid, maleic acid, citric acid, isocitric acid, and ascorbic acid. Among these, acetic acid, propionic acid, and butyric acid are preferred, and acetic acid and propionic acid are more preferred. The organic acid having two or more carbon atoms may be used alone or in combination. Although there is no particular upper limit on the number of carbon atoms in the organic acids, from the perspective of solubility in water, those with eight or fewer carbon atoms are preferable, six or fewer carbon atoms are more preferable, and three or fewer carbon atoms are most preferable. The organic acid having two or more carbon atoms may also be a salt of such an organic acid.

Solution B, which is treated in the second step, preferably has a pH of 0 to 7, more preferably a pH of 0.5 to 5, and most preferably a pH of 1 to 3. The concentration of component X in solution B is preferably 0.1 g/L to 100 g/L, more preferably 1 g/L to 70 g/L, and most preferably 10 g/L to 50 g/L. When solution B obtained in the first step does not have the above-mentioned preferred pH or concentration of component X, the pH of said solution B may be adjusted or solution B may undergo pretreatment, such as ultrafiltration, prior to the second step.

In the second step, in addition to solution B, examples of solutions that can be used for liquid flow when treating solution B with a weakly basic anion exchange resin include deionized water, and an eluent. Deionized water is used to elute impurities that are not adsorbed by the weakly basic anion exchange resin. The eluent is used to elute substances adsorbed by the weakly basic anion exchange resin in this step.

In this step, solution B, deionized, and an eluent are preferred as solutions that can be used for elution when treating solution B with weakly basic anion exchange resin.

((2-1) Step for Passing Solution B Obtained by First Step Through Weakly Basic Anion Exchange Resin to Remove at Least a Portion of Neutral Impurities That Do Not Adsorb Onto Weakly Basic Anion Exchange Resin)

As a condition for passing solution B obtained by the first step through the weakly basic anion exchange resin, the flow rate is preferably 0.1 to 5 SV, more preferably 0.2 to 4 SV, and most preferably 0.5 to 2 SV.

The liquid flow temperature of the weakly basic anion exchange resin is preferably 3°C or higher, 5°C or higher, 8°C or higher, or 10°C or higher, and is preferably 30°C or lower, 25°C or lower, 20°C or lower, 15°C or lower, 14°C or lower, 13°C or lower, or 12°C or lower. These upper and lower limits may be combined arbitrarily. In other embodiments, the temperature is 3 to 30°C, more preferably 3 to 25°C, even more preferably 3 to 20°C, still more preferably 3 to 15°C, still more preferably 5 to 15°C, and most preferably 8 to 15°C.

Through this step (2-1), at least a portion of the neutral impurities are eluted from the weakly basic anion exchange resin, and components other than the neutral impurities in solution B are adsorbed onto the weakly basic anion exchange resin. The components other than the neutral impurities include component X.

### ((2-2) Step for Eluting at Least a Portion of Impurities Remaining After 2-1) from Weakly Basic Anion Exchange Resin)

In order to elute the impurities remaining after 2-1) from the weakly basic anion exchange resin, the eluent preferably contains at least one of water, ammonium hydroxide, sodium hydroxide, and potassium hydroxide, etc., and more preferably contains water and sodium hydroxide.

The concentration of basic substances such as ammonium hydroxide, sodium hydroxide, and potassium hydroxide in the eluent is preferably 0.0000 mol/L or more, and in order of preference, 0.0001 mol/L or more, 0.0005 mol/L or more, 0.001 mol/L or more, 0.002 mol/L or more, and 0.005 mol/L or more; and in order of preference, 0.5 mol/L or less, 0.1 mol/L, and 0.05 mol/L or less. These upper and lower limits may be combined arbitrarily. In other embodiments, it is preferably 0.0000 to 0.5 mol/L, 0.0001 to 0.5 mol/L, 0.0005 to 0.5 mol/L, 0.001 to 0.5 mol/L, or 0.005 to 0.5 mol/L.

In this specification, the amount of solution passing through the ion exchange resin is expressed as the resin volume (the volume ratio of the solution when the resin volume of the ion exchange resin is 1, hereinafter denoted as "RV").

The flow rate of the eluent is preferably 0.1 to 5 SV, more preferably 0.2 to 4 SV, and still more preferably 0.5 to 2 SV. The amount of eluent is preferably 0.5 to 20 RV, more preferably 1 to 15 RV, still more preferably 2 to 12 RV, and most preferably 3 to 10 RV.

The liquid flow temperature of the weakly basic anion exchange resin is preferably 3°C or higher, and in order of preference, 5°C or higher, 8°C or higher, 20°C or higher, 25°C or higher, and 30°C or higher; and preferably 60°C or lower, and in order of preference, 50°C or lower, 40°C or lower, 30°C or lower, and 25°C or lower. These upper and lower limits may be combined arbitrarily. The above liquid flow temperature is also preferably 3 to 60°C, more preferably 20 to 50°C, still more preferably 20 to 40°C, particularly preferably 20 to 40°C, and most preferably 20 to 25°C.

In a more preferred embodiment, after passing water, a basic aqueous solution such as ammonium hydroxide, sodium hydroxide, or potassium hydroxide is passed.

Through this step (2-2), at least a portion of the impurities remaining in the weakly basic anion exchange resin after step (2-1) is washed away. Furthermore, by setting the temperature within the aforementioned ranges, there is a tendency to reduce the content of component V in the final fermented product and to increase the content ratio of component X.

### ((2-3) Step for Separately Eluting Acidic Compounds Containing Component X and Impurities Remaining After 2-2) from Weakly Basic Anion Exchange Resin)

In order to separately elute the acidic compounds containing component X and the impurities remaining after step 2-2) from the weakly basic anion exchange resin, the eluent preferably contains at least one of water, ammonium hydroxide, sodium hydroxide, potassium hydroxide, etc., and more preferably contains sodium hydroxide.

The concentration of basic substances such as ammonium hydroxide, sodium hydroxide, and potassium hydroxide in the eluent is preferably 0.001 mol/L or more, and in order of preference, 0.005 mol/L or more, 0.01 mol/L or more, 0.02 mol/L or more, and 0.03 mol/L or more, and in order of preference, 2.0 mol/L or less, 1.0 mol/L, and 0.5 mol/L or less. These upper and lower limits may be combined arbitrarily. In other embodiments, the concentration is preferably 0.001 to 2.0 mol/L, 0.005 to 1.0 mol/L, 0.001 to 1.0 mol/L, 0.001 to 0.5 mol/L, 0.01 to 0.5 mol/L, 0.02 to 0.5 mol/L, or 0.03 to 0.5 mol/L.

The flow rate of the eluent is preferably 0.1 to 5 SV, more preferably 0.2 to 4 SV, and still more preferably 0.5 to 2 SV. The amount of eluent is preferably 0.5 to 20 RV, more preferably 1 to 15 RV, still more preferably 2 to 12 RV, and most preferably 3 to 10 RV.

The liquid flow temperature of the weakly basic anion exchange resin is preferably 3°C or higher, more preferably 5°C or higher, even more preferably 8°C or higher, still more preferably 10°C or higher, and particularly preferably 15°C or higher, and preferably 40°C or lower, more preferably 30°C or lower, still more preferably 25°C or lower, even more preferably 20°C or lower, and particularly preferably 15°C or lower. These upper and lower limits may be combined arbitrarily. The above liquid flow temperatures are also preferably, in the following order of preference: 3 to 40°C, 3 to 30°C, 3 to 25°C, 3 to 20°C, 3 to 15°C, 5 to 20°C, 8 to 20°C, 10 to 20°C, and 15 to 20°C.

By setting the liquid flow temperature of the weakly basic anion exchange resin within the aforementioned ranges, isomerization from aldoses to ketoses can be prevented or reduced, thereby reducing the content of isomers of sialylated oligosaccharides of component X and tending to improve the content ratio of component X.

Solution C obtained in the second step preferably has a pH of 5 to 12, more preferably 6 to 10, and still more preferably 7 to 9. The concentration of component X in solution C is preferably 0.1 g/L to 100 g/L, more preferably 1 g/L to 70 g/L, even more preferably 3 g/L to 50 g/L, and most preferably 5 g/L to 20 g/L.

### (Third Step)

In the third step the pH of solution C is adjusted to obtain solution D.

In other words, in the third step, the pH of solution C obtained in the second step, that is, solution C eluted from the weakly basic anion exchange resin in the second step, is adjusted. Solution C obtained in the second step is basic due to ion exchange with the weakly basic anion exchange resin. Therefore, more specifically, solution D is obtained by adjusting the pH of solution C to preferably 0 to 6, more preferably 1 to 5, and most preferably 2 to 4. In this case, it is preferable to perform the pH adjustment of the third step without mixing solution C separated in the second step. Since the pH adjustment in the third step improves the adsorption capacity of the weakly basic anion exchange resin, treating solution D with the weakly basic anion exchange resin in the fourth step described below allows for improved separation of component X from other impurities.

Known methods may be used to adjust the pH of solution C, and examples include adding inorganic acids, specifically hydrochloric acid, sulfuric acid, nitric acid, or phosphoric acid, to solution C, or passing solution C through a cation exchange resin. Preferably, the method involves passing solution C through a cation exchange resin, and more preferably, it involves passing solution C through a weakly acidic cation exchange resin.

The cation exchange resin used in the third step is not particularly limited, and examples include ion exchange resins having carboxylate groups as exchange groups of the weakly acidic cation exchange resin. Furthermore, examples of matrix materials for the cation exchange resin include porous types, macroporous types, gel types, styrene types, and acrylic types, etc. Specific examples of cation exchange resins include, for example, WK10, WK10S, WK11, WT01S, WK40, WK60, and WK100 manufactured by DIAION, PUROLITE (registered trademark) C104, C106, C107E, and C115E manufactured by PUROLITE, AMBERLITE (e.g., FPC76J, FPC3500, etc.) manufactured by DUPONT DE NEMOURS, INC., the DIAION (registered trademark) WK Series (e.g., WK10, WK100, WK10S, WK11, WK40, WK60, and WK60L, etc.) manufactured by MITSUBISHI CHEMICAL CORPORATION, and WT01S manufactured by MITSUBISHI CHEMICAL CORPORATION, and LEWATIT CNP80WS manufactured by LANXESS PTE. LTD.

As the cation exchange resin used in the third step, it is more preferable that the cation exchange resin has carboxylate groups as exchange groups and that the matrix material of the cation exchange resin is of a porous type. Specifically, examples include PUROLITE C115E manufactured by PUROLITE.

Furthermore, the ionic form of the carboxylate groups in the cation exchange resin is not particularly limited, and examples include H⁺, Na⁺, K⁺, and NH₄⁺, and H⁺ is preferred.

The pH of solution D obtained in the third step is preferably 0 to 6, more preferably 1 to 5, and most preferably 2 to 4. The concentration of component X in solution D is preferably 0.1 g/L to 100 g/L, more preferably 1 g/L to 70 g/L, even more preferably 3 g/L to 50 g/L, and most preferably 5 g/L to 20 g/L. When solution D obtained in the third step does not have the above-mentioned preferred pH or concentration of component X, a treatment such as adjustment of the pH of solution D may be performed after the third step.

In the third step, in addition to solution C, examples of solutions that can be used for liquid flow when treating solution C with a cation exchange resin include deionized water. Deionized water is used to elute substances that do not adsorb to the acidic cation exchange resin, thereby improving the recovery rate of component X.

In this step, solution C and deionized water are preferred as solutions that can be used for elution when treating solution C with a cation exchange resin.

As liquid flow conditions for treating solution C with a cation exchange resin, the flow rate is preferably SV 0.1 to 5, more preferably SV 0.2 to 4, and most preferably SV 0.5 to 2. Under these liquid flow conditions, the temperature is preferably 30°C or less, more preferably 25°C or less, and still more preferably 20°C or less. Specifically, the temperature is preferably 3 to 30°C, more preferably 5 to 25°C, and still more preferably 8 to 20°C.

An eluate that has passed through the cation exchange resin, that is, solution D, is recovered and used in the fourth step described below.

### (Fourth Step)

In the fourth step, solution D is treated with a weakly basic anion exchange resin to obtain solution E.

Specifically, in the fourth step, by treating solution D obtained in the third step with a weakly basic anion exchange resin, component X is separated from acidic impurities other than component X, and solution E which contains component X at a high concentration is obtained. Examples of acidic impurities other than component X include component Y, organic acids other than component Y, and component Z, etc.

The weakly basic anion exchange resin used in the fourth step may be one included as an example of the weakly basic anion exchange resin used in the second step. In particular, in the fourth step, a free form is preferred as the ionic state of the tertiary amino group exchange groups of the weakly basic anion exchange resin.

Solution E obtained in the fourth step preferably has a pH of 5 to 12, more preferably 6 to 10, and still more preferably 7 to 9. The concentration of component X in solution E is preferably 0.1 g/L to 100 g/L, more preferably 1 g/L to 70 g/L, even more preferably 3 g/L to 50 g/L, and most preferably 5 g/L to 20 g/L. When solution E obtained in the fourth step does not have the above-mentioned preferred pH or concentration of component X, a treatment such as adjustment of the pH of solution E may be performed after the fourth step. Furthermore, the pH of solution E may be adjusted to make it acidic.

In the fourth step, in addition to solution D, examples of solutions that can be used for liquid flow when treating solution D with a weakly basic anion exchange resin include deionized water, and an eluent. Deionized water is used to elute substances that do not adsorb to the weakly basic ion exchange resin, thereby improving the recovery rate of component X.

In this step, solution D and deionized water are preferred as solutions that can be used for elution when treating solution D with a weakly basic anion exchange resin.

As liquid flow conditions for passing solution D through a weakly basic anion exchange resin, the flow rate is preferably SV 0.1 to 5, more preferably SV 0.2 to 4, and most preferably SV 0.5 to 2.5. Under these liquid flow conditions, the temperature is preferably 30°C or less, more preferably 25°C or less, and still more preferably 20°C or less. Specifically, the temperature is preferably 3 to 30°C, more preferably 5 to 25°C, and still more preferably 8 to 20°C.

Furthermore, component X has lower acidity than components Y and Z, or exhibits weaker adsorption to the weakly basic anion exchange resin. When solution D is passed through the weakly basic anion exchange resin, component X tends to elute first, followed by components Y and Z. Therefore, it is preferable to recover a fraction immediately following an inflection point in electrical conductivity just before the elution of component X ends as a fraction containing a large amount of component X and little of components Y and Z, it is more preferable to recover a fraction from an inflection point in electrical conductivity just before the elution of component X ends up to 1.0 RV, it is still more preferable to recover a fraction from an inflection point in electrical conductivity just before the elution of component X ends to 0.5 RV, and it is most preferable to recover a fraction from an inflection point in electrical conductivity just before the elution of component X ends to 0.1 RV. The recovered solution is a fermented product containing component X at a high concentration.

In this specification, an inflection point in electrical conductivity refers to a point on a graph with the liquid volume passed through the weakly basic anion exchange resin on the horizontal axis and electrical conductivity on the vertical axis, where the curve changes from concave down to concave up, or vice versa.

### (Fifth Step)

In the fifth step, solution E is powderized to obtain a fermented product.

Solution E used in the fifth step may be a solution obtained after the fourth step by steps of concentration, decolorization, and/or filtration described below. Known methods may be used for powderization, such as freeze-drying or spray drying, and spray drying is preferred. Furthermore, the fermented product may be obtained as solution E or as a solution obtained after the concentration, decolorization, filtration, etc., described below, without powderizing the fermented product.

### (Concentration, Decolorization, and Filtration)

After the fourth step, solution E (fermented product) recovered in the fourth step may be subjected to steps for concentration, decolorization, and/or filtration. Known methods may be used for concentration, decolorization, and/or filtration. Furthermore, the order of concentration, decolorization, and filtration is not critical.

### (Concentration and Filtration)

E.g., examples include a process involving fine filtration of solution E obtained in step 4 using a membrane with a pore size of 0.2 to 1.2 µm, specifically, a method for treating solution E obtained in step 4, or a solution obtained by treating solution E with activated carbon, using an MF membrane.

Furthermore, a microfiltered solution may be concentrated under reduced pressure to concentrate component X in the solution to a concentration of 10 to 600 g/L, more preferably 100 to 550 g/L, and still more preferably 300 to 500 g/L.

### (Decolorization)

The concentrated solution may be subjected to a decolorization treatment by adding activated carbon.
Specific examples of the activated carbon used here include, for example, CARBORAFFIN, KYORYOKU SHIRASAGI, SEISEI SHIRASAGI, TOKUSEI SHIRASAGI, SHIRASAGI A, SHIRASAGI C, SHIRASAGI ANOX-1, SHIRASAGI FAC-10, SHIRASAGI WP-H, SHIRASAGI DO-2, SHIRASAGI DO-5, GRANULAR SHIRASAGI G2C, GRANULAR SHIRASAGI WH2C, GRANULAR SHIRASAGI W2C, GRANULAR SHIRASAGI WH5C, GRANULAR SHIRASAGI W5C, GRANULAR SHIRASAGI LGK-100, GRANULAR SHIRASAGI LGK-400, GRANULAR SHIRASAGI KL, GRANULAR SHIRASAGI LH2C, SPHERICAL SHIRASAGI X8100H, SPHERICAL SHIRASAGI XS8100H, GRANULAR SHIRASAGI G2X, GRANULAR HAKURO G5X, GRANULAR HAKURO S2X, GRANULAR HAKURO WH2X, GRANULAR HAKURO X2M, GRANULAR HAKURO C2C, GRANULAR HAKURO C2X, SPHERICAL HAKURO X7000H, SPHERICAL HAKURO X7100H, SPHERICAL HAKURO XS7100H, SPHERICAL SHIRASAGI X7000H-3, SPHERICAL SHIRASAGI X7100H-3, SPHERICAL SHIRASAGI LGK-700, SPHERICAL SHIRASAGI DX7-3, SHIRASAGI M, SHIRASAGI P, GRANULAR SHIRASAGI GM2X, and ZAITSU AKSJ manufactured by OSAKA GAS CHEMICALS CO., LTD., TAIKO S, TAIKO K, TAIKO P, TAIKO W, TAIKO A, and TAIKO Y manufactured by FUTAMURA CHEMICAL CO., LTD., ALTAPRINT 246, ALTAPYNE PITCH, AQUAGUARD, AQUANUCHAR, KRAFTSPERSE 1251, NUCHAR, NUCHAR ACS 310X-2L, NUCHAR BAX 1100LD, NUCHAR BAX 1500, NUCHAR BAX 1700, NUCHAR BAX LBE, NUCHAR FUELSORB, NUCHAR HCA LBE, NUCHAR RGC POWDER, NUCHAR SA, NUCHAR SA PCL, NUCHAR SA-20, NUCHAR SA-T, NUCHAR SN, NUCHAR SQUARE HCA, NUCHAR STANDARD HCA, NUCHAR WV-A 1100 8X25, NUCHAR WV-A 1100 8X35, NUCHAR WV-A 1500 10X25, and NUCHAR WV-B 30 GRANULAR manufactured by INGEVITY CORPORATION, HR manufactured by RIGHT SOLUTION PUBLIC COMPANY LIMITED, and GW-110, GW-120, GW-520, PW-100, PW-003, PW-210, PS-003, PS-250, PS-252, PG-100, PG-001, GG-770, GG-771, GG-772, GG-773, GG-775, GG-730, GG-731, GG-732, GG-733, GG-735, GD-120, GD-200, and PD-005 manufactured by SUN FRONTIER CHEMICAL CO., LTD., etc., and Taiko Y is preferred.

Furthermore, by subjecting a decolorized filtrate obtained from the above process to ultrafiltration using an ultrafiltration membrane (hereinafter also referred to as a "UF membrane") capable of removing substances with a molecular weight of 6000 or more (MWCO = 6000), for example, it is possible to remove endotoxins, residual proteins, high-molecular-weight peptides, toxins, and the like from the decolorized filtrate. The ultrafiltrated solution can be concentrated under reduced pressure to adjust the concentration of component X in the solution to a concentration of 10 to 600 g/L, more preferably 100 to 550 g/L, and still more preferably 300 to 500 g/L.

### (Fermentation Product Obtained by Second Step)

Furthermore, the method for producing a fermented product may include the first step and the second step, and not include the third step and the fourth step.

The fermented product obtained by the second step includes the solutions obtained by the first and second steps, as well as the solutions or powders obtained by concentrating, decolorizing, filtering, and/or powderizing the solutions with the aforementioned methods.

Furthermore, the ratio (content of component Z1 / content of component X) of the content of component Z1 relative to the content of component X in the fermented product obtained by the second step is preferably 0.03 or less, more preferably 0.01 or less, and still more preferably 0.001 or less, on a mass basis. Specific examples of neutral sugars constituting component Z1 include glucose and/or lactose.

Furthermore, the ratio (content of component Z2 / content of component X) of the content of component Z2 relative to the content of component X in the fermented product obtained by the second step is preferably 0.5 or less, and more preferably 0.4 or less, on a mass basis.

Examples of other impurities contained in the fermented product obtained by the second step include Cl⁻, PO₄³⁻, and SO₄²⁻, etc., as inorganic anions, as well as sodium ions, potassium ions, ammonium ions, etc., as inorganic cations.

The fermented product obtained through the first to fifth steps described in the [1. Fermented Product] section of this specification has a higher content ratio of component X and lower contents of components Y and Z compared to the fermented product obtained through the first and second steps.

### EXAMPLES

The following describes preferred aspects of the present invention, but the present invention is not limited thereto.

Unless otherwise specified, percentages represent mass%.

### [Analytical Examples]

In the examples, the analysis and quantification of each component was performed according to the following procedure.

### <Analysis Example 1-1: Measurement of Content and Content Ratio of Sialic Acids or Sialylated Oligosaccharides (Component X) from Solid Matter>

### (Preparation of Component X Standard Solution)

A component X standard solution was prepared by accurately weighing approximately 0.1 g of the following standard material, dissolving it in distilled water to a concentration of 1 g/L, and then diluting it with a diluent (solution prepared by mixing mobile phase A and mobile phase B of the HPLC-CAD system in a 6:4 volume ratio) to achieve component X concentrations of 0.04 g/L, 0.05 g/L, and 0.06 g/L.
- Standard: 3SL·Na, manufactured by KYOWA HAKKO BIO CO., LTD.; purity: 99.2% (as 3SL·Na)
- Standard: 6SL·Na, manufactured by KYOWA HAKKO BIO CO., LTD.; purity: 97.0% (as 6SL·Na)

### (Preparation of Sample Solution)

The fermented product was a solid, such as a powder, and the sample solution was prepared by accurately weighing approximately 0.5 g of the solid, dissolving it in distilled water, and diluting it with a diluent. Note that when the peak area value of component X in the sample solution fell outside the range of the calibration curve, the sample solution was adjusted with the diluent as appropriate to ensure that the concentration of component X fell within the range of the calibration curve.

### (Analysis of Component X)

The prepared sample solution and the component X standard solution were analyzed under the following conditions, the peaks for component X (retention times: NeuAc 16.0 min, 3SL 21.4 min, 6SL 22.5 min) were detected, a calibration curve was constructed using the three-point calibration method based on the peak area values of component X in the standard solution, and the concentration of component X in the sample solution was then quantified.

### (Analytical Conditions for Component X)

Component X was analyzed by HPLC-CAD under the following conditions.

Analytical columns:
- Guard column: HILICpak VN-50G 4A, inner diameter 4.6 mm, length 10 mm, particle size 5 µm, Cat. No. F6711400 (manufactured by SHOWA DENKO KABUSHIKI KAISHA)
- Column: HILICpak VN-50 4D, inner diameter 4.6 mm, length 150 mm, particle size 5 µm, Cat. No. F7630500 (manufactured by SHOWA DENKO KABUSHIKI KAISHA)
- Column temperature: Constant at approximately 60°C
- Mobile phase: Mobile phase A: Acetonitrile; Mobile phase B: 25 mM ammonium formate
- Mobile Phase Flow Rate (Flow Velocity): 1.0 mL/min
- Detector: Charged Aerosol Detector (CAD)
- CAD drying temperature: 35°C

The gradient was performed under the conditions shown in Table 1 below.

**[Table 1]**

| Gradient Method | | | |
|---|---|---|---|
| Time (min) | Mobile Phase A Liquid (%) | Mobile Phase B Liquid (%) | Mobile Phase Flow Rate (mL/min) |
| 0.0 | 90 | 10 | 1.0 |
| 24.0 | 72 | 28 | 1.0 |
| 30.0 | 62 | 38 | 1.0 |
| 35.0 | 62 | 38 | 1.0 |
| 37.0 | 90 | 10 | 1.0 |
| 50.0 | 90 | 10 | 1.0 |

### (Calculation of Component X Content Ratio from Solids)

When C is defined as the slope of the calibration curve calculated from the peak area value of component X in the obtained component X standard solution, D is the intercept, E is the moisture content (mass%) of the sample calculated by the Karl Fischer titration method in Analysis Example 7, F is the concentration of fermented products in the sample solution (g/L), and G is the peak area value of the sample solution, the content ratio of component X was calculated using the following Equation (1a). 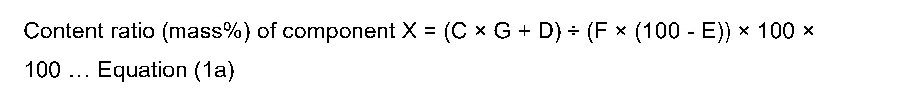 Content ratio (mass%) of component X = (C × G + D) ÷ (F × (100 - E)) × 100 × 100 ... Equation (1a)

### <Analysis Example 1-2: Calculation of Content Ratio of Component X from Solution>

### (Calculation of Content of Other Impurities Detected by HPLC)

Analysis was performed under the same conditions as in Analysis Example 1-1 using a component X standard solution and a sample solution containing 1 g/L of component X, and other impurities detected by HPLC were quantified using a one-point calibration method based on the peak area value of component X in the component X standard solution. In this instance, the other impurities detected by HPLC refer to components detected by HPLC other than components X, Y, Z, W, and V. The sample solution containing 1 g/L of component X was prepared based on the content of component X determined in Analysis Example 1-1. When R is defined as the peak area value of the component X standard solution and S is the peak area value of other impurities detected by HPLC in the sample solution, the content of other impurities detected by HPLC in the sample was calculated using the following Equation (13a). 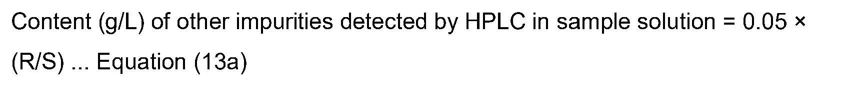 Content (g/L) of other impurities detected by HPLC in sample solution = 0.05 × (R/S) ... Equation (13a)

### (Calculation of Content of Other Impurities Detected by HPLC in Fermented Product / Content of Component X)

Since the content of other impurities detected by HPLC in the sample solution calculated by Equation (13a) corresponds to the content of other impurities detected by HPLC in a sample solution containing 1 g/L of component X, the content of other impurities detected by HPLC in the fermented relative to the content of component X in the fermented product was calculated using the following Equation (13b). 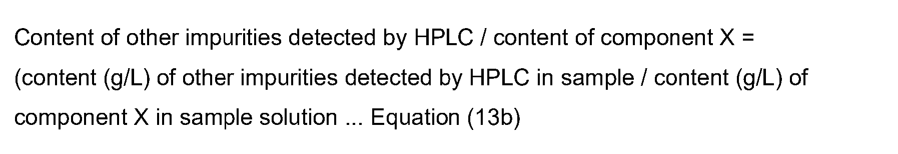 Content of other impurities detected by HPLC / content of component X = (content (g/L) of other impurities detected by HPLC in sample / content (g/L) of component X in sample solution ... Equation (13b)

(Calculation of Total Amount of (Content of Other Impurities Detected by HPLC in Fermented Product / Content of Component X))

When n is defined as the total number of impurities detected in a sample, the total amount U₂ of the ratio of the content of individual impurities relative to the content of component X is calculated using Equation (13c) below. In Equation (13c) below, U is the ratio of the content of an individual impurity detected by HPLC relative to the content of component X.
[Equation 1] ${U}_{2} = {\sum }_{i = 1}^{n} Ui$ Equation (13c)

### (Calculation of Component X Content Ratio from Solution)

When Y is defined as the ratio of the acetic acid content relative to the content of component X (specifically, sialyllactose) detected in the sample; Z₁ is the ratio of the neutral sugar (specifically, lactose) content relative to the content of component X, Z₂ is the ratio of the acidic sugar (specifically, NeuAc) content relative to the content of component X, W is the ratio of the content of component W relative to the content of component X, V is the ratio of the content of component V relative to the content of component X, M₁ is the ratio of the content of inorganic metals relative to the content of component X, U₃ is the moisture content when the solution is solidified using SD, or the like, I₁ is the total amount of the ratio of the content of each impurity relative to the content of component X in the sample solution, I₂ is the total amount of the ratio of the content of each impurity relative to the content of component X in the sample solution on a wet basis, X₁ is the purity of component X in the sample solution on a wet basis, and X₂ is the purity of component X in the sample solution, the purity of component X in the sample solution can be calculated using the following equations (14a) to (14d). 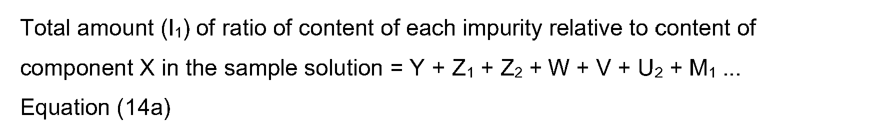 Total amount (I1) of ratio of content of each impurity relative to content of component X in the sample solution = Y + Z1 + Z2 + W + V + U2 + M1 ... Equation (14a) 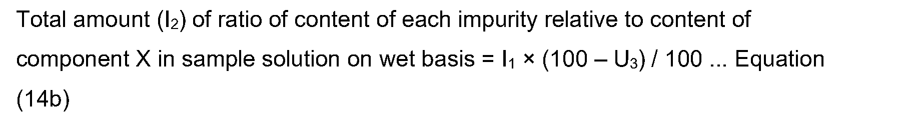 Total amount (I2) of ratio of content of each impurity relative to content of component X in sample solution on wet basis = I1 × (100 - U3) / 100 ... Equation (14b) 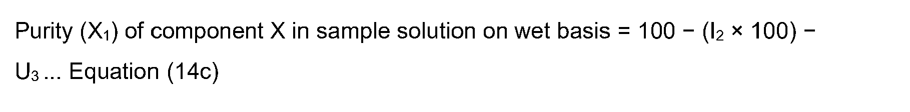 Purity (X1) of component X in sample solution on wet basis = 100 - (I2 × 100) - U3 ... Equation (14c) $Content ratio \left({X}_{2}\right) of component X in sample solution = {X}_{1} \times 100 / \left(100-{U}_{3}\right)$ Equation (14d)

### (Calculation of Component X Content)

When the fermented product is a solid such as a powder, the content of component X was calculated using the following Equation (1b). Note that the definitions of C, G, and D in Equation (1b) are the same as those in Equation (1a). $Content \left(g/g\right) of component X = \left(C\times G+D\right) \div F$ Equation (1b)

Furthermore, when the fermented product is a solution and the content ratio of component X is calculated using the solution as a sample without evaporating and drying the fermented product, the fermented product is analyzed as the sample solution, and when the peak area value of the resulting sample solution is defined as G', and the sample solution is diluted to calculate G, the dilution factor is defined as y, and the content (g/L) of component X in the fermented product was calculated using Equation (1c). Note that the definitions of C and D in Equation (1c) are the same as those in Equation (1a). $Content \left(g/L\right) of component X = \left(C\times G′+D\right) \times y$ Equation (1c)

### <Analysis Example 2: Measurement of Acetic Acid (Component Y) Content>

### (Preparation of Acetic Acid Standard Solution)

An acetic acid standard solution was prepared by accurately weighing approximately 0.14 g of sodium acetate (FUJIFILM WAKO PURE CHEMICAL CORPORATION, Product No. 198-15965) as a standard, dissolving it in water, and adjusting the concentration to approximately 0.1 g/L.

### (Analysis of Acetic Acid (Component Y))

Analysis was performed under the following conditions using a 0.1 g/L acetic acid standard solution and a sample solution having a component X content of approximately 10 g/L, an acetic acid peak (retention time: acetic acid 34.7 min) was detected, and component Y (i.e., acetic acid) was quantified using a one-point calibration method based on the peak area value of the standard. The sample solution containing approximately 10 g/L of component X was prepared based on the component X content calculated in Analysis Example 1-1.

### (Analytical Conditions for Acetic Acid (Component Y))

The quantification of acetic acid was performed by HPLC-UV under the following conditions.
- Guard column: SUGAR SH-G, inner diameter 6.0 mm, length 50 mm, particle size 10 µm, P/N: F6700080 (manufactured by SHOWA DENKO KABUSHIKI KAISHA)
- Column: SUGAR SH1011, inner diameter 8.0 mm, length 300 mm, particle size 6 µm (two connected columns), P/N: F6378100 (manufactured by SHOWA DENKO KABUSHIKI KAISHA)
- Column temperature: Constant at approximately 60°C
- Mobile phase: 5 mM H₂SO₄
- Mobile Phase Flow Rate: 0.6 mL/min
- Detector: UV-Vis absorption detector (SPD-20A, manufactured by SHIMADZU CORPORATION)
- Detection wavelength: 210 nm
- Limit of Detection: 0.650 mg/L
- Limit of Quantitation: 1.97 mg/L

### (Calculation of Component Y Content)

When A is defined as the peak area value of the acetic acid standard solution and B is the peak area value of the sample solution, the acetic acid concentration in the sample solution was calculated using the following Equation (2a). $Acetic acid content \left(g/L\right) in sample solution = 0.1 \times \left(B/A\right)$ Equation (2a)

### (Calculation of Content of Component Y / Content of Component X in Fermented Product)

Since the acetic acid content in the sample solution calculated by Equation (1) corresponds to the content of component Y in a sample solution containing 10 g/L of component X, the content of component Y relative to the content of component X (content of component Y / content of component X) in the fermented product was calculated using the following Equation (2b). 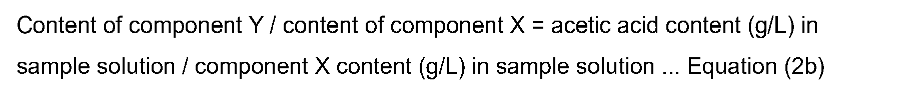 Content of component Y / content of component X = acetic acid content (g/L) in sample solution / component X content (g/L) in sample solution ... Equation (2b)

### <Analysis Example 3: Analysis of Lactose (Component Z1)>

### (Preparation of Lactose Standard Solution)

Lactose monohydrate (FUJIFILM WAKO PURE CHEMICAL CORPORATION, 128-00095) was used as the standard for the quantitative analysis of lactose (component Z1) by HPLC.

The lactose standard solution was prepared by accurately weighing approximately 0.11 g of the lactose standard and dissolving it in distilled water to achieve a lactose concentration of 0.025 g/L.

### (Calculation of Component Z1 Content)

Analysis was performed under the following conditions using the lactose standard solution and a sample solution containing approximately 1 g/L of component X, and component Z1 was quantified using a one-point calibration method based on the peak area value of lactose in the lactose standard solution. The sample solution containing approximately 1 g/L of component X was prepared based on the component X content calculated in Analysis Example 1-1. When J is defined as the peak area value of the lactose standard solution and K is the peak area value of the sample solution; the lactose content in the sample solution was calculated using the following Equation (3a). $Z 1 \left(lactose\right) content \left(g/L\right) in sample solution = 0.025 \times \left(K/J\right)$ Equation (3a)

### (Analytical Conditions for Lactose (Z1))

The quantification of lactose was performed by HPLC-PAD under the following conditions.

| | |
|---|---|
| - Guard column: | Dionex CarboPac PA1 IC Standard Bore Guard Column, inner diameter 4 mm, length 50 mm, particle size 10 µm, Cat. No. 043096 (THERMO FISHER SCIENTIFIC INC.) |
| - Column: | Dionex CarboPac PA1 IC Standard Analytical Column, inner diameter 4 mm, length 250 mm, particle size 10 µm, Cat. No. 035391 (THERMO FISHER SCIENTIFIC INC.) |
| - Column temperature: | Constant at approximately 30°C |
| - Mobile phase: | Mobile phase A: Distilled water; Mobile phase B: 0.5 mol/L NaOH; Mobile phase C: 0.28 M Na acetate + 0.03 M NaOH |
| - Mobile Phase Flow Rate: | 1.0 mL/min |
| - Detector: | Pulse amperometric detector (PAD) |
| - Limit of Detection: | 0.451 mg/L |
| - Limit of Quantitation: | 1.37 mg/L |

The gradient was performed under the conditions shown in Table 2 below.

**[Table 2]**

| Gradient Method | | | |
|---|---|---|---|
| Time (min) | Mobile Phase A Liquid (%) | Mobile Phase B Liquid (%) | Mobile Phase C Liquid (%) |
| 0 | 72 | 28 | 0 |
| 5 | 72 | 28 | 0 |
| 13.5 | 50 | 28 | 22 |
| 20 | 50 | 28 | 22 |
| 38 | 0 | 35 | 65 |
| 50 | 0 | 35 | 65 |
| 50 | 72 | 28 | 0 |

### (Calculation of Content of Component Z1 / Content of Component X in Fermented Product)

Since the content of Z1 in the sample solution calculated in Equation (3a) corresponds to the content of component Z1 in a sample solution containing 1 g/L of component X, the content of lactose (component Z1) relative to the content of component X in the fermented product was calculated using the following Equation (3b). 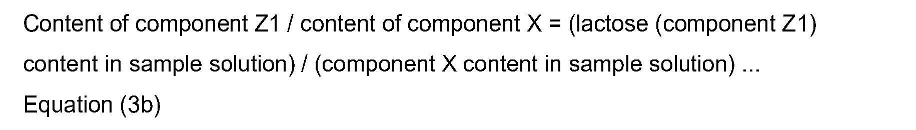 Content of component Z1 / content of component X = (lactose (component Z1) content in sample solution) / (component X content in sample solution) ... Equation (3b)

Furthermore, even when component Z1 is glucose, the content of component Z1 / the content ratio of component X in the fermented product can be calculated in the same manner as in the aforementioned Analysis Example 3.

### <Analysis Example 4: Analysis of NeuAc (Component Z2)>

### (Preparation of NeuAc Standard Solution)

N-acetylneuraminic acid dihydrate (MA167834 manufactured by BIOSYNTH CARBOSYNTH) was used as the standard for the quantitative analysis of NeuAc (component Z2) by HPLC.

A NeuAc standard solution was prepared by accurately weighing approximately 0.11 g of the NeuAc standard, dissolving it in distilled water to a concentration of 1 g/L, and then diluting it with a diluent (solution prepared by mixing mobile phase A and mobile phase B of the HPLC-CAD system in a 6:4 volume ratio) to achieve a NeuAc concentration of 0.04 g/L.

### (Calculation of Component Z2 Content)

Analysis was performed under the same conditions as in Analysis Example 1-1 using the NeuAc standard solution and a sample solution containing 1 g/L of component X, and component Z2 was quantified using a one-point calibration method based on the NeuAc peak area value of the NeuAc standard solution. The sample solution containing 1 g/L of component X was prepared based on the content of component X determined in Analysis Example 1-1. When L is defined as the peak area value of the NeuAc standard and M is the NeuAc peak area value of the sample solution, the NeuAc content in the sample was calculated using the following Equation (4a). $Z 2 \left(NeuAc\right) content \left(g/L\right) in sample solution = 0.04 \times \left(M/L\right)$ Equation (4a)

### (Calculation of Content of Component Z2 / Content of Component X in Fermented Product)

Since the content of Z2 in the sample solution calculated in Equation (4a) corresponds to the content of component Z2 in a sample solution containing 1 g/L of component X, the content of NeuAc (component Z2) relative to the content of component X in the fermented product was calculated using the following Equation (4b). 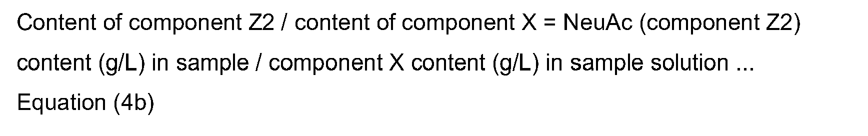 Content of component Z2 / content of component X = NeuAc (component Z2) content (g/L) in sample / component X content (g/L) in sample solution ... Equation (4b)

### <Analysis Example 5: Analysis of Isomers of Component X (Component W)>

### (Calculation of Component W Content)

Analysis was performed under the same conditions as in Analysis Example 1-1 using the component X standard solution and a sample solution containing 1 g/L of component X, and component W was quantified using a one-point calibration method based on the component X peak area value of the component X standard solution. The sample solution containing 1 g/L of component X was prepared based on the content of component X determined in Analysis Example 1-1. When N is defined as the component X peak area value of the component X standard solution and O is the component W peak area value of the sample solution, the component W content in the sample was calculated using the following Equation (11a). 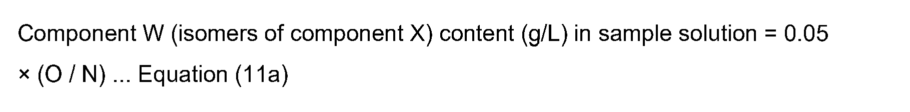 Component W (isomers of component X) content (g/L) in sample solution = 0.05 × (O / N) ... Equation (11a)

### (Calculation of Content of Component W / Content of Component X in Fermented Product)

Since the content of W in the sample solution calculated in Equation (11a) corresponds to the content of component W in a sample solution containing 1 g/L of component X, the content of isomers of component X (component W) relative to the content of component X in the fermented product was calculated using the following Equation (11b). 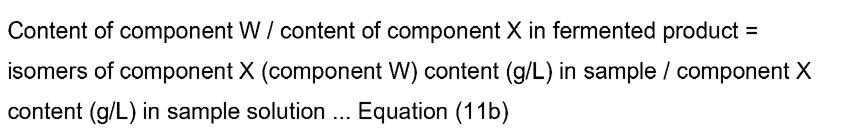 Content of component W / content of component X in fermented product = isomers of component X (component W) content (g/L) in sample / component X content (g/L) in sample solution ... Equation (11b)

### <Analysis Example 6: Analysis of Component V>

### (Calculation of Component V Content)

Analysis was performed under the same conditions as in Analysis Example 1-1 using the component X standard solution and a sample solution containing 1 g/L of component X, and component V was quantified using a one-point calibration method based on the component X peak area value of the component X standard solution. The sample solution containing 1 g/L of component X was prepared based on the content of component X determined in Analysis Example 1-1. When P is defined as the component X peak area value of the component X standard solution and Q is the component V peak area value of the sample solution, the component V content in the sample was calculated using the following Equation (12a). $Component V content \left(g/L\right) in sample solution = 0.05 \times \left(P/Q\right)$ Equation (12a)

### (Calculation of Content of Component V / Content of Component X in Fermented Product)

Since the content of component V in the sample solution calculated in Equation (12a) corresponds to the content of component V in a sample solution containing 1 g/L of component X, the content of component V relative to the content of component X in the fermented product was calculated using the following Equation (12b). 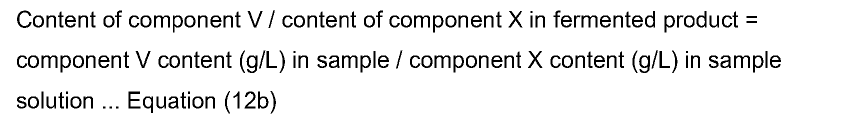 Content of component V / content of component X in fermented product = component V content (g/L) in sample / component X content (g/L) in sample solution ... Equation (12b)

### <Analysis Example 7: Measurement of Moisture Content in Fermentation Product>

The moisture content (mass%, moisture value of E in Equation (1a)) in the fermented product was analyzed using the Karl Fischer titration method under the following conditions.
- Test method: JP17 <2.48> (Moisture Determination Method in The Japanese Pharmacopoeia, Seventeenth Edition)
- Amount added: 0.1 g of powder
- Titrant: Aquamicron (registered trademark) Titrant SS-Z 3 mg (Karl Fischer Reagent SS-Z)
- Dehydrating Solvent: Aquamicron (registered trademark) Dehydrating Solvent ML (General Purpose)
- Dissolution time: ~5 min

The present invention will be described in detail below with reference to examples; however, the present invention is not limited to the following examples as long as the essence of the invention is not lost.

In this specification, 3SL refers to 3-sialyllactose, and the sodium salt of 3SL, 3'-sialyllactose sodium salt, is also referred to as 3SL·Na.

In addition, 6SL refers to 6-sialyllactose, and the sodium salt of 6SL, 6'-sialyllactose sodium salt, is also referred to as 6SL·Na.

### [Experimental Example 1: Production of Fermented Product High in 3'-Sialyllactose (3SL)]

### [Preparation Example 1] <Fermentative Production of 3SL>

The culture broth was prepared by fermenting 3SL using recombinant Escherichia coli according to the methods described in International Publication No. 2015/037698 and Japanese Patent Application Laid-Open Publication No. 2008-5794, and thereafter, the culture was heat-treated at 70°C for 40 minutes to inactivate the bacterial cells, and sulfuric acid was added to adjust the pH to 4.3 ± 0.3. Subsequently, the fermentation broth was treated by the inactivated bacterial cells (biomass) being separated from the fermentation solution using a cross-flow filter (0.1 µm filter), and the culture supernatant (hereinafter referred to as solution A) was obtained. In this instance, solution A was obtained from two lots, lot 1 and lot 2. Table 3 lists the composition of the obtained solution A.

### [Comparative Example 1] <Purification of 3SL via First and Second Steps>

As Comparative Example 1, 0.355 L of solution A from lot 1 (10°C), obtained in [Preparation Example 1] above, was used in the ion-exchange chromatography of the first and second steps.

In the first step, 0.11 L of TG-Gel (also known as XUS 40232.01 (manufactured by THE DOW CHEMICAL COMPANY)), which is an H⁺ type strongly acidic cation exchange resin, was used, solution A was passed through the TG-Gel at an SV of 0.86, causing cationic impurities in solution A to be adsorbed onto the strongly acidic cation exchange resin, thereby yielding solution B in which the cationic impurities had been removed from solution A.

In this specification, the term "treatment solution" refers to a solution used in the first to fifth steps, etc., to obtain a fermented product containing a high proportion of component X. Furthermore, in Examples 1 to 4 and Comparative Examples 1 and 2, unless otherwise specified, RV and SV denote the volume ratios of the solution when the resin volume of the second column A845/1967 is 1.

Next, solution B was treated with 0.142 L of PUROLITE (registered trademark) A845/1967 (manufactured by PUROLITE), an OH⁻ type weakly basic anion exchange resin, as the resin of the second step. First, solution B was passed through this ion exchange resin at an SV of 0.83 at 10°C to adsorb the anionic impurities and 3SL present in solution B onto the weakly basic anion exchange resin. Subsequently, 3.2 RV of deionized water at 25°C was passed through at an SV of 0.83 to remove neutral substances that do not adsorb onto the weakly basic anion exchange resin by washing. Thereafter, the anionic impurities adsorbed onto the weakly basic anion exchange resin were removed by passing 2.3 RV of a 0.005 mol/L NaOH aqueous solution at 25°C through the resin at an SV of 0.83. Then a 0.035 mol/L NaOH aqueous solution at 10°C was passed through at an SV of 0.83, and the 3SL adsorbed onto the weakly basic anion exchange resin was eluted as a sodium salt, and when the solution was collected with 4.65 RV as the endpoint (as shown in fig. 1), 0.66 L of eluate (solution C) was obtained. During elution, the eluate was fractionated every 0.155 RV, and the composition of the eluate was analyzed.

The results of the analysis of the composition of this eluate are shown in fig. 1. In fig. 1, the horizontal axis shows the liquid flow volume in the second step as the "second column liquid flow volume", and the vertical axis shows the content and electrical conductivity of each component. Electrical conductivity is indicated as Cond.

Furthermore, Table 3 shows the composition of solution C obtained in the second step in the column for Comparative Example 1. Note that the composition of solution C obtained in the second step was calculated cumulatively from the line graph in fig. 1, which was derived by analyzing the eluates fractionated during the treatment in the second step. Although 3SL is also present in the eluate as 3SL·Na, the 3SL content and percentage listed in Table 3 are values converted to 3SL (free form).

### [Example 1] <Purification of 3SL via First to Fourth Steps>

As Example 1, the treatments of the first to fourth steps were performed using 0.355 L of solution A from lot 2 (10°C), obtained in [Preparation Example 1] above. The first to fourth steps were performed as described above in Comparative Example 1 except that in Step 2, 2.9 RV of deionized water at 25°C was passed through at an SV of 0.83, and then 2.8 RV of a 0.005 mol/L NaOH aqueous solution at 25°C was passed through at an SV of 0.83, and the solution C obtained in the second step was passed in the third step, and solution C was obtained by treating solution A from lot 2 obtained in [Preparation Example 1].

Solution C obtained in the second step was continuously passed through PUROLITE (registered trademark) C115E and PUROLITE (registered trademark) A845/1967 in the third and fourth steps in the following manner.

In the third step, 0.08 L of solution C obtained in the second step was passed through the H⁺ type weakly acidic cation exchange resin PUROLITE (registered trademark) C115E (manufactured by PUROLITE) while cooling to 10°C, yielding solution D adjusted to a pH of 2 to 4. Solution C was passed through PUROLITE (registered trademark) C115E at an SV of 1.5 relative to when the resin volume of the third column of PUROLITE (registered trademark) C115E is 1.

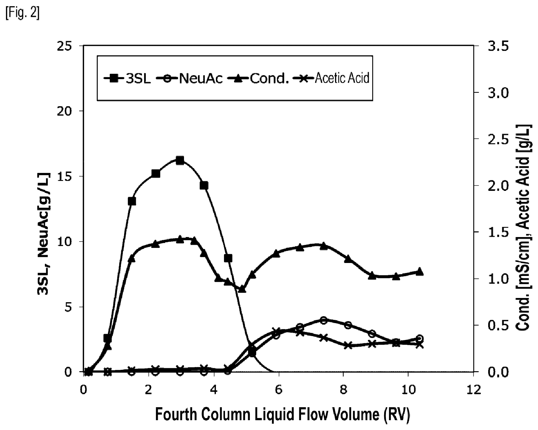

In the fourth step, 0.051 L of solution D obtained in the third step was passed through PUROLITE (registered trademark) A845/1967 (manufactured by PUROLITE), which is an OH⁻ type weakly basic anion exchange resin, while cooling to 10°C, to remove analogous substances, specifically acidic impurities, and the solution was collected at the endpoint of 5.8 RV shown in fig. 2, yielding 0.82 L of eluate (solution E). Solution D was passed through PUROLITE (registered trademark) A845/1967 at an SV of 2.3 relative to when the resin volume of the fourth column of PUROLITE (registered trademark) A845/1967 is 1, the eluate was collected every 0.148 RV, and the composition of the eluate was analyzed.

The results of the analysis of the composition of solution E are shown in fig. 2. In fig. 2, the horizontal axis shows the liquid flow volume in the fourth step as the "fourth column liquid flow volume", and the vertical axis shows the content and electrical conductivity of each component. Electrical conductivity is indicated as Cond.

Furthermore, Table 3 shows the composition of solution E obtained in the fourth step in the column for Example 1.

In Table 3, the [g/L] value for 3SL is calculated using Equation (1c) of Analysis Example 1-1 above, the [g/L] value for NeuAc is calculated using Equation (4a) of Analysis Example 4 above, and the [g/L] value for acetic acid is calculated using Equation (2a) of Analysis Example 2 above.

In Table 3, the "Yield" for Example 1 is a value calculated using Equation (5a) below, and the "Yield" for Comparative Example 1 is a value calculated using Equation (5b) below. 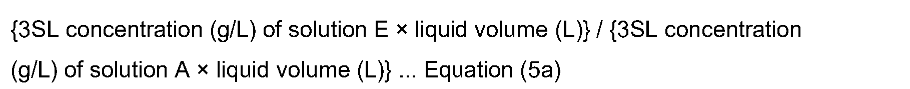 {3SL concentration (g/L) of solution E × liquid volume (L)} / {3SL concentration (g/L) of solution A × liquid volume (L)} ... Equation (5a) 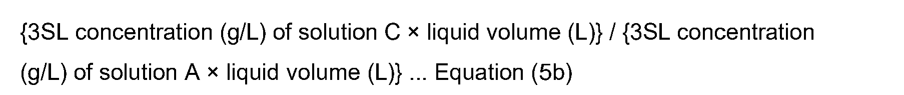 {3SL concentration (g/L) of solution C × liquid volume (L)} / {3SL concentration (g/L) of solution A × liquid volume (L)} ... Equation (5b)

The term "yield" in the examples corresponds to a value defined in this specification as "the ratio (total mass of component X in solution E / total mass of component X in solution A) of the total mass of component X in solution E relative to the total mass of component X in solution A".

In Table 3, the "residual rate" of acetic acid in Example 1 is a value calculated using Equation (9a) below, and the "residual rate" of acetic acid in Comparative Example 1 is a value calculated using Equation (9a) below. 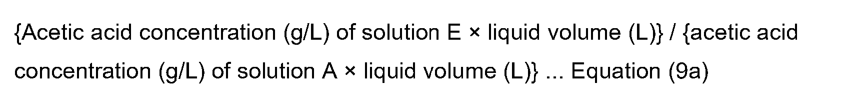 {Acetic acid concentration (g/L) of solution E × liquid volume (L)} / {acetic acid concentration (g/L) of solution A × liquid volume (L)} ... Equation (9a) 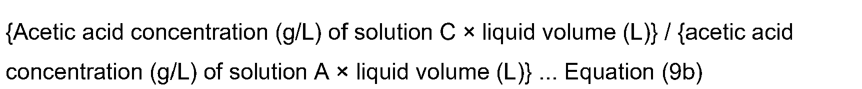 {Acetic acid concentration (g/L) of solution C × liquid volume (L)} / {acetic acid concentration (g/L) of solution A × liquid volume (L)} ... Equation (9b)

The term "residual rate" of acetic acid in Example 1 corresponds to a value defined in this specification as "the ratio (total mass of component Y in solution E / total mass of component Y in solution A) of the total mass of component Y in solution E relative to the total mass of component Y in solution A".

**[Table 3]**

| Lot | | Resin | Sample | 3S L | Liquid Volume | Yield | NeuAc | | Acetic Acid | | |
|---|---|---|---|---|---|---|---|---|---|---|---|
| | | | | [g/L] | [L] | | [g/L] | [Ratio to 3SL] | [g/L] | [Ratio to 3SL] | Residual Rate |
| Lot 1 | Comparative Example 1 | NA | Solution A | 25.9 | 0.355 | NA | 12.0 | 0.462 | 2.86 | 0.110 | NA |
| | | 2 Columns | Solution C | 11.5 | 0.660 | 0.82 | 0.809 | 0.0706 | 0.138 | 0.0120 | 0.090 |
| Lot 2 | Example 1 | NA | Solution A | 25.1 | 0.355 | NA | 10.7 | 0.428 | 2.81 | 0.112 | NA |
| | | 4 Columns | Solution E | 8.94 | 0.819 | 0.82 | 0.159 | 0.0178 | 0.0432 | 0.00483 | 0.035 |

| | | | | | | | | | | | |
|---|---|---|---|---|---|---|---|---|---|---|---|
| NA = not applicable | | | | | | | | | | | |

Fig. 1 shows that in Comparative Example 1, acetic acid is eluted when the liquid flow volume of the second column is at approximately 3 RV or higher, and that acetic acid is eluted even while 3SL is being eluted. On the other hand, fig. 2 shows that in Example 1, the amount of acetic acid eluted while 3SL is being eluted is lower than that shown in fig. 1.

As shown in Table 3, the (content of component Z2 (NeuAc) / the content of component X (3SL)) (the "[Ratio to 3SL] of NeuAc" in Table 3) in solution E (i.e., the fermented product of the present invention) obtained in Example 1 was less than 0.02. Furthermore, the (content of component Y (acetic acid) / the content of component X (3SL)) (the "[Ratio to 3SL] of Acetic Acid" in Table 3) was less than 0.010. In contrast, in solution C obtained in Comparative Example 1, the (content of component Z2 (NeuAc) / content of component X (3SL)) exceeded 0.02. In addition, the (content of component Y (acetic acid) / the content of component X (3SL)) (the "[Ratio to 3SL] of Acetic Acid" in Table 3) exceeded 0.010.

### [Experimental Example 2: Production of Fermented Product High in 6'-Sialyllactose (6SL)]

### [Preparation Example 2] <Fermentative Production of 6SL>

The culture broth was prepared by fermenting 6SL using recombinant Escherichia coli according to the methods described in International Publication No. 2015/037698 and Japanese Patent Application Laid-Open Publication No. 2008-5794, and the culture was heat-treated at 70°C for 40 minutes to inactivate the bacterial cells, and sulfuric acid was added to adjust the pH to 4.3 ± 0.3. Subsequently, the inactivated bacterial cells (biomass) were separated from fermentation solution using a cross-flow filter (0.1 µm filter), and the culture supernatant (solution A) was obtained. The obtained solution A is shown in Table 4.

### [Comparative Example 2] <Purification of 6SL via First and Second Steps>

As Comparative Example 2, 51.9 kL of the industrial-scale solution A obtained in [Preparation Example 2] above was used in the ion exchange chromatography of the first and second steps.

In the first step, 17 kL of the same H⁺ type strongly acidic cation exchange resin (TG-Gel) as in Example 1 and Comparative Example 1 was used, solution A (8.0°C) was passed through the TG-Gel at an SV of 1.0, causing cationic impurities in solution A to be adsorbed onto the strongly acidic cation exchange resin, thereby yielding solution B in which the cationic impurities had been removed from solution A.

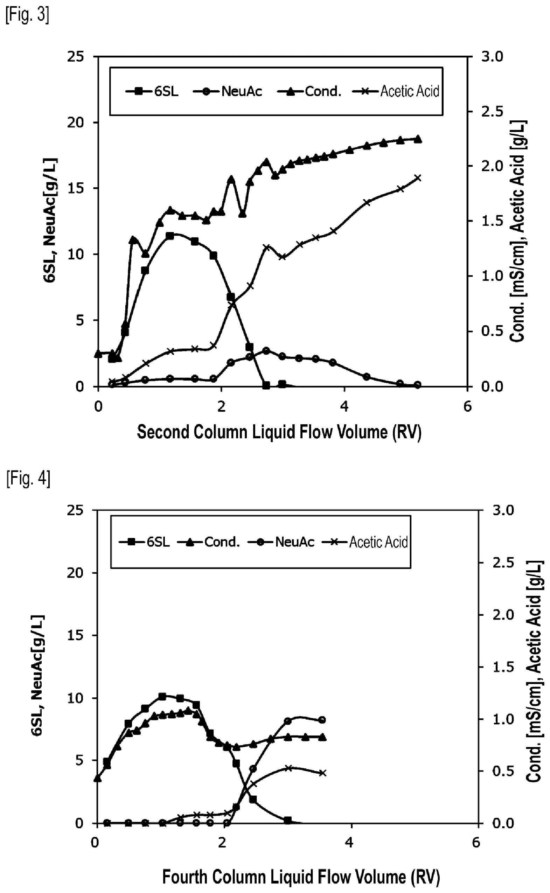

Next, 22 kL of PUROLITE (registered trademark) A845/1967 (manufactured by PUROLITE), the same OH⁻ type weakly basic anion exchange resin as that used in Example 1 and Comparative Example 1, was used as the resin of the second step. Solution B was passed through this weakly basic anion exchange resin at an SV of 0.73 at 11.1°C to adsorb the anionic impurities and 6SL present in solution B onto the weakly basic anion exchange resin. Subsequently, 3.6 RV of deionized water at 10.0°C was passed through at an SV of 0.81 to remove neutral substances that do not adsorb onto the weakly basic anion exchange resin by washing from the weakly basic anion exchange resin. Furthermore, the anionic impurities adsorbed onto the weakly basic anion exchange resin were removed by passing 2.6 RV of a 0.005 mol/L NaOH aqueous solution at 10.0°C through the resin at an SV of 0.82. Then a 0.035 mol/L NaOH aqueous solution at 10.0°C was passed through at an SV of 0.62, and the 6SL adsorbed onto the weakly basic anion exchange resin was eluted as a sodium salt. The eluate was fractionated every 0.1 to 0.2 RV, and after analyzing the composition of each fractionated solution, 60.0 kL of eluate (solution C) was obtained by collecting the solution at the endpoint of 2.73 RV in fig. 3. During elution, the eluate was fractionated every 15 minutes, and the composition of the eluate was analyzed.

The results of the analysis of the composition of this fractionated eluate are shown in fig. 3. In fig. 3, the horizontal axis shows the liquid flow volume in the second step as the "second column liquid flow volume", and the vertical axis shows the content and electrical conductivity of each component. Electrical conductivity is indicated as Cond.

Furthermore, Table 4 shows the composition of the eluate obtained in the second step in the column for Comparative Example 2. Note that the composition of solution C of Comparative Example 2 in Table 4 was calculated cumulatively from the line graph in fig. 3, which was derived by analyzing the compositions of the fractionated eluates during the treatment in the second step. Although 6SL is also present in the eluate as 6SL·Na, the 6SL content and percentage listed in Table 4 are values converted to 6SL (free form).

### [Example 2] <Purification of 6SL via First to Fourth Steps>

As Example 2, the eluate from the second step (solution C) obtained in [Comparative Example 2] was passed through the third step, and the eluate from the third step (solution D) was passed through the fourth step, thereby performing the processes of the first to fourth steps.

The eluate obtained in the second step was continuously passed through PUROLITE (registered trademark) C115E and PUROLITE (registered trademark) A845/1967 in the third and fourth steps in the following manner.

In the third step, 12 kL of the eluate obtained in the second step (solution C) was passed through the H⁺ type weakly acidic cation exchange resin PUROLITE (registered trademark) C115E (manufactured by PUROLITE) while cooling to 11.0°C, yielding solution D adjusted to a pH of 2 to 4. Solution C was passed through PUROLITE (registered trademark) C115E at an SV of 1.4 relative to when the resin volume of the third column of PUROLITE (registered trademark) C115E is 1.

In the fourth step, 9.5 kL of solution D obtained in the third step was passed through PUROLITE (registered trademark) A845/1967 (manufactured by PUROLITE), which is an OH⁻ type weakly basic anion exchange resin, while cooling to 8.0°C, to remove analogous substances, specifically acidic impurities, and the solution was collected at the endpoint of 2.69 RV shown in fig. 4, yielding 54.0 kL of eluate (solution E). Solution D was passed through PUROLITE (registered trademark) A845/1967 at an SV of 1.6 relative to when the resin volume of the fourth column of PUROLITE (registered trademark) A845/1967 is 1, the eluate was collected every 15 minutes, and the composition of the eluate was analyzed.

The results of the analysis of the composition of solution E are shown in fig. 4. In fig. 4, the horizontal axis shows the liquid flow volume in the fourth step as the "fourth column liquid flow volume", and the vertical axis shows the content and electrical conductivity of each component. Electrical conductivity is indicated as Cond.

Furthermore, Table 4 shows the composition of solution E obtained in the fourth step in the column for Example 2.

In Table 4, the [g/L] value for 6SL is calculated using Equation (1c) of Analysis Example 1-1 above, the [g/L] value for NeuAc is calculated using Equation (4a) of Analysis Example 4 above, and the [g/L] value for acetic acid is calculated using Equation (2a) of Analysis Example 2 above.

Other than the fact that 3SL was replaced with 6SL, the "Yield" and "Residual Rate" of acetic acid in Table 4 are values that were calculated using the same method as the "Yield" and "Residual Rate" of acetic acid in Table 3.

**[Table 4]**

| Lot | | Resin | Sample | 6SL | Liquid Volume | Yield | NeuAc | | Acetic Acid | | |
|---|---|---|---|---|---|---|---|---|---|---|---|
| | | | | [g/L] | [kL] | | [g/L] | [Ratio to 6SL] | [g/L] | [Ratio to 6SL] | Residual Rate |
| Lot 3 | NA | NA | Solution A | 12.6 | 51.9 | NA | 2.17 | 0.172 | 6.37 | 0.505 | NA |
| | Comparative Example 2 | 2 Columns | Solution C | 6.93 | 60.0 | 0.64 | 0.870 | 0.126 | 0.406 | 0.0586 | 0.074 |
| | Example 2 | 4 Columns | Solution E | 7.35 | 54.0 | 0.61 | 0.00925 | 0.00126 | 0.0440 | 0.00598 | 0.0072 |

| | | | | | | | | | | | |
|---|---|---|---|---|---|---|---|---|---|---|---|
| NA = not applicable | | | | | | | | | | | |

Fig. 3 shows that in Comparative Example 2, acetic acid elutes immediately after the start of the second step, and continues to elute even while 6SL is being eluted. On the other hand, fig. 4 shows that in Example 2, the amount of acetic acid eluted while 6SL is being eluted is lower than that in Comparative Example 2.

As shown in Table 4, the (content of component Z2 (NeuAc) / the content of component X (6SL)) (the "[Ratio to 6SL] of NeuAc" in Table 4) in solution E (i.e., the fermented product of the present invention) obtained in Example 2 was less than 0.02. Furthermore, the (content of component Y (acetic acid) / the content of component X (6SL)) (the "[Ratio to 6SL] of Acetic Acid" in Table 4) was less than 0.010. In contrast, in solution C obtained in Comparative Example 2, the (content of component Z2 (NeuAc) / content of component X (3SL)) exceeded 0.02, and the (content of component Y (acetic acid) / the content of component X (3SL)) (the "[Ratio to 3SL] of Acetic Acid" in Table 4) exceeded 0.010.

### [Example 3] <Purification of 3SL and Preparation of Powder Containing 3SL>

3SL was fermented using recombinant Escherichia coli according to the methods described in International Publication No. 2015/037698 and Japanese Patent Application Laid-Open Publication No. 2008-5794, the culture was heat-treated at 70°C for 40 minutes to inactivate the bacterial cells, and sulfuric acid was added to adjust the pH to 4.3 ± 0.3, and as a result, 78.7 kL of culture broth with the composition described as the sample "A. Broth" in Tables 5 and 6 was obtained. Subsequently, the inactivated bacterial cells (biomass) were separated from fermentation solution using a cross-flow filter (0.1 µm filter), and a total of 128 kL of the culture supernatant (solution A) was obtained. The 3SL concentration, [Ratio to 3SL] of NeuAc, and [Ratio to 3SL] of acetic acid of solution A are as shown in Tables 5 and 6.

Next, the obtained solution A was divided into two portions, and the first to fourth steps were performed using the same procedure as in Examples 1 and 2, as described below.

The first portion of the resulting solution A (64.1 kL) and the second portion (63.9 kL) were used separately in the first and second steps, respectively.

In the first step, 17 kL of H⁺ type TG-Gel (manufactured by THE DOW CHEMICAL COMPANY), which is a strongly acidic cation exchange resin, was used, solution A at 8.0°C was passed through TG-Gel at an SV of 0.95 in the strongly acidic cation exchange resin for the first portion, and solution A at 8.0°C was passed through TG-Gel at an SV of 1.0 in the strongly acidic cation exchange resin for the second portion, causing cationic impurities in solution A to be adsorbed onto the strongly acidic cation exchange resin, thereby yielding two solutions B in which the cationic impurities had been removed from solution A.

Next, 23 kL of PUROLITE (registered trademark) A845/1967 (manufactured by PUROLITE), which is an OH⁻ type weakly basic anion exchange resin, was used as the resin of the second step, and in order to ensure that the anionic impurities and 3SL in solution B were adsorbed by this weakly basic anion exchange resin, solution B was passed through the weakly basic anion exchange resin by passing solution B at 8.9°C through the weakly basic anion exchange resin for the first portion at an SV of 0.72, and passing solution B at 8.7°C through the weakly basic anion exchange resin for the second portion at an SV of 0.74. Subsequently, 3.1 RV of deionized water at 10.0°C was passed through the weakly basic anion exchange resin for the first portion and 3.1 RV of deionized water at 10.0°C was passed through the weakly basic anion exchange resin for the second portion both at an SV of 0.78 to remove neutral substances that do not adsorb onto the weakly basic anion exchange resin from the weakly basic anion exchange resin. Subsequently, the anionic impurities adsorbed onto the weakly basic anion exchange resin were removed by passing 2.7 RV of a 0.005 mol/L NaOH aqueous solution at 10.0°C through weakly basic anion exchange resin for the first portion, and 2.6 RV of a 0.005 mol/L NaOH aqueous solution at 10.0°C through the weakly basic anion exchange resin for the second portion, respectively, at an SV of 0.78. Subsequently, a 0.035 mol/L NaOH aqueous solution at 10.0°C was passed through the weakly basic anion exchange resin for the first portion, and a 0.035 mol/L NaOH aqueous solution at 10.0°C was passed through the weakly basic anion exchange resin for the second portion, respectively, at an SV of 0.77, and the 3SL adsorbed onto the weakly basic anion exchange resin was eluted as a sodium salt, yielding two solutions C.

Next, as the third step, the two solutions C obtained in the second step were each passed through 11 kL of the H⁺ type weakly acidic cation exchange resin PUROLITE (registered trademark) C115E (manufactured by PUROLITE), yielding two solutions D adjusted to a pH range of 2 to 4. Note that solutions C were each passed through PUROLITE (registered trademark) C115E at an SV of 1.6 relative to when the resin volume of the third column of PUROLITE (registered trademark) C115E is 1 while cooling to 11.1°C and 11.0°C, respectively.

Then, as the fourth step, the two solutions D obtained in the third step were each passed through 10 kL of PUROLITE (registered trademark) A845/1967 (manufactured by PUROLITE), which is an OH⁻ type weakly basic anion exchange resin, while cooling to 8.0°C, yielding 85.0 kL of eluate (solution E) for the first portion and 80.5 kL of eluate (solution E) for the second portion. Note that solutions D were each passed through PUROLITE (registered trademark) A845/1967 at an SV of 1.8 relative to when the resin volume of the fourth column of PUROLITE (registered trademark) A845/1967 is 1.

The pH of the two solutions E obtained in the fourth step was adjusted to 5.5 to 6.1 by adding 0.5 mol/L hydrochloric acid to each. Subsequently, each pH-adjusted solution E was passed through a 1.2 µm in-line filter (MF membrane) and a 0.45 µm in-line filter (MF membrane) in this order, yielding two treated solutions. Next, each of the treated solutions was concentrated until the SL·Na concentration reached 400 g/L, and the two concentrated solutions were mixed to obtain a single concentrated solution.

Next, activated carbon (Taiko (registered trademark) Y) manufactured by FUTAMURA CHEMICAL CO., LTD. was added to this concentrate to adsorb and remove colored components, and the like, in the concentrate. Additionally, immediately prior to proceeding to a filtration process, 0.5 mol/L hydrochloric acid was added to the concentrate containing the activated carbon to adjust the pH to 5.3 to 6.2. This pH-adjusted concentrate was passed through a 0.45 µm in-line filter (MF membrane) to remove fine activated carbon and foreign matter as part of the filtration process, then it was passed through a UF membrane (6000 Da cut-off), thereby removing endotoxins, etc., from the concentrate. Subsequently, the permeate obtained by passing through the UF membrane was concentrated until the SL·Na concentration reached approximately 400 g/L, and a sterilized treated liquid (liquid fermented product) was obtained by passing through a 0.2 µm in-line filter.

Subsequently, as a fifth step, the sterilized treated solution was spray-dried using a spray dryer, yielding spray-dried powder (SD) as the fermented product. This yielded 2281 kg of powder with an SL purity of 88 mass%, a NeuAc content of 0.00552 in terms of ratio to 3SL, and an acetic acid content of 0.000473 in terms of ratio to 3SL, as shown in the "SD" columns of Tables 5 and 6.

Tables 5 and 6 show the results of the analysis of the compositions of solution A, solution E, and SD obtained in Example 3. In Tables 5 and 6, the [g/L] value for 3SL is calculated using Equation (1c) of Analysis Example 1-1 above, the [g/L] value for NeuAc is calculated using Equation (4a) of Analysis Example 4 above, and the [g/L] value for acetic acid is calculated using Equation (2a) of Analysis Example 2 above.

The "yield" in Table 5 is a value calculated using the following Equation (10). 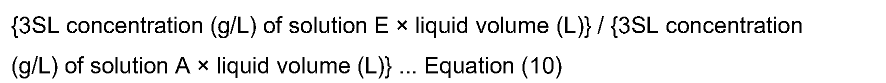 {3SL concentration (g/L) of solution E × liquid volume (L)} / {3SL concentration (g/L) of solution A × liquid volume (L)} ... Equation (10)

The "3SL purity" in Table 5 is a value that corresponds to the "content ratio of component X per unit of solid matter in the fermented product" in this specification. The "3SL purity" was calculated using the method described in Equation (1a) of Analysis Example 1-1 above.

The [ratio to 3SL] of acetic acid in Table 5 is a value that corresponds to "the ratio (content of component Y / content of component X) of the content of component Y relative to the content of component X in solution E" on a mass basis in the present specification, and in cases in which the sample is SD, corresponds to "the ratio (content of component Y / content of component X) of the content of component Y relative to the content of component X in the fermented product" on a mass basis in the present specification. This value was calculated using Equation (2b) in Analytical Example 2 above.

The [ratio to 3SL] of NeuAc in Table 6 is a value that corresponds to "the ratio (content of component Z2 / content of component X) of the content of each compound of component Z2 relative to the content of component X in the fermented product" on a mass basis in the present specification. This value was calculated using Equation (4b) in Analytical Example 4 above.

The "residual rate" of acetic acid in Table 5 was calculated in the same manner as the "residual rate" of acetic acid in Example 1.

In this powder, glucose and lactose, which are neutral sugars (Z1), had a ratio to 3SL (the ratio (content of component Z1 / content of component X) of the content of each compound of component Z1 relative to the content of component X in the fermented product) below the detection limit.

**[Table 5]**

| Lot | Resin | Sample | 3S L | Liquid Volume | Yield | Moisture Content | 3SL Purity | Powder Quantity | Acetic Acid | | |
|---|---|---|---|---|---|---|---|---|---|---|---|
| | | | [g/L] | [kL] | | [%] | [%] | [kg] | [g/L] | [Ratio to 3SL] | Residual Rate |
| Lot 4 | NA | A. Broth | 39.2 | 78.7 | NA | NA | NA | NA | 1.00 | 0.0255 | NA |
| Lot 4 First Portion | NA | Solution A First Portion | 23. 6 | 64.1 | NA | NA | NA | NA | 0.45 0 | 0.0191 | NA |
| Lot 4 Second Portion | NA | Solution A Second Portion | 21. 8 | 63.9 | NA | NA | NA | NA | 0.60 0 | 0.0275 | NA |
| Lot 4 First Portion | 4 Columns | Solution E First Portion | 12. 5 | 85.0 | 0.7 0 | NA | NA | NA | 0.06 24 | 0.00501 | 0.18 |
| Lot 4 Second Portion | 4 Columns | Solution E Second Portion | 12. 3 | 80.5 | 0.7 1 | NA | NA | NA | ND* | ≤ 0.000047 6** (ND*) | 0.0 |
| Lot 4 | NA | SD | NA | NA | NA | 8.2 | 88 | 2281 | NA** * | 0.000473 | NA |

| | | | | | | | | | | | |
|---|---|---|---|---|---|---|---|---|---|---|---|
| NA = not applicable, ND = not detected * Detection limit of 0.000476 g/L for sample solution ** Value when analyzing a sample solution prepared with component X = 3SL at 10 g/L *** 0.00473 g/L when analyzing a sample solution prepared with component X = 3SL at 10 g/L | | | | | | | | | | | |

**[Table 6]**

| Lot | Resin | Sample | Glucose | | Lactose | | NeuAc | |
|---|---|---|---|---|---|---|---|---|
| | | | [g/L] | [Ratio to 3SL] | [g/L] | [Ratio to 3SL] | [g/L] | [Ratio to 3SL] |
| Lot 4 | NA | A. Broth | NA | NA | NA | NA | 15.4 | 0.392 |
| Lot 4 First Portion | NA | Solution A First Portion | NA | NA | NA | NA | 9.10 | 0.386 |
| Lot 4 Second Portion | NA | Solution A Second Portion | NA | NA | NA | NA | 8.80 | 0.403 |
| Lot 4 First Portion | 4 Columns | Solution E First Portion | NA | NA | NA | NA | 0.0725 | 0.00582 |
| Lot 4 Second Portion | 4 Columns | Solution E Second Portion | NA | NA | NA | NA | 0.0711 | 0.00579 |
| Lot 4 | NA | SD | ND*** | ≤ 0.000467* (ND***) | ND**** | ≤ 0.000451* (ND*) | NA***** | 0.00552 |

| | | | | | | | | |
|---|---|---|---|---|---|---|---|---|
| NA = not applicable, ND = not detected * Value when analyzing a sample solution prepared with component X = 3SL at 1 g/L ** Value when analyzing a sample solution prepared with component X = 3SL at 10 g/L *** Detection limit of 0.000467 g/L for sample solution **** Detection limit of 0.000451 g/L for sample solution ***** 0.0552 g/L when analyzing a sample solution prepared with component X = 3SL at 1 g/L | | | | | | | | |

### [Example 4] <Purification of 6SL and Preparation of Powder Containing 6SL>

6SL was fermented using recombinant Escherichia coli according to the methods described in International Publication No. 2015/037698 and Japanese Patent Application Laid-Open Publication No. 2008-5794, the culture was heat-treated at 70°C for 40 minutes to inactivate the bacterial cells, and sulfuric acid was added to adjust the pH to 4.3 ± 0.3, and as a result, 83.4 kL of culture broth with the composition described as the sample "A. Broth" in Tables 7 and 8 was obtained. Subsequently, the inactivated bacterial cells (biomass) were separated from the fermentation solution using a cross-flow filter (0.1 µm filter), and a total of 121 kL of the culture supernatant (solution A) was obtained. The 6SL concentration, [Ratio to 6SL] of NeuAc, and [Ratio to 6SL] of acetic acid of solution A are as shown in Tables 7 and 8.

Next, the obtained solution A was divided into two portions, and the first to fourth steps were performed using the same procedure as in Examples 1 and 2, as described below.

The first portion of the resulting solution A (60.8 kL) and the second portion (60.0 kL) were used separately in the first and second steps, respectively.

In the first step, 17 kL of TG-Gel (manufactured by THE DOW CHEMICAL COMPANY), which is an H⁺ type strongly acidic cation exchange resin, was used, solution A at 8.0°C was passed through TG-Gel at an SV of 1.0 in the strongly acidic cation exchange resin for the first portion, and solution A at 8.0°C was passed through TG-Gel at an SV of 1.0 in the strongly acidic cation exchange resin for the second portion, causing cationic impurities to be adsorbed onto the strongly acidic cation exchange resin, thereby yielding two solutions B in which the cationic impurities had been removed from solution A.

Next, 22 kL of PUROLITE (registered trademark) A845/1967 (manufactured by PUROLITE), which is an OH⁻ type weakly basic anion exchange resin, was used as the resin of the second step, and in order to ensure that the anionic impurities and 6SL were adsorbed by this weakly basic anion exchange resin, solution B was passed through the weakly basic anion exchange resin by passing solution B at 12.3°C through the weakly basic anion exchange resin for the first portion at an SV of 0.77, and passing solution B at 14.0°C through the weakly basic anion exchange resin for the second portion at an SV of 0.77. Subsequently, 3.1 RV of deionized water at 10.0°C was passed through the weakly basic anion exchange resin for the first portion and 3.6 RV of deionized water at 10.0°C was passed through the weakly basic anion exchange resin for the second portion respectively at an SV of 0.79 and 0.70 to remove neutral substances that do not adsorb onto the weakly basic anion exchange resin from the weakly basic anion exchange resin. Subsequently, the anionic impurities adsorbed onto the weakly basic anion exchange resin were removed by passing 3.1 RV of a 0.005 mol/L NaOH aqueous solution at 10.0°C through weakly basic anion exchange resin for the first portion, and 2.9 RV of a 0.005 mol/L NaOH aqueous solution at 10.0°C through the weakly basic anion exchange resin for the second portion, respectively, at an SV of 0.82 and 0.70. Subsequently, a 0.035 mol/L NaOH aqueous solution at 10.0°C was passed through the weakly basic anion exchange resin for the first portion, and a 0.035 mol/L NaOH aqueous solution at 10.0°C was passed through the weakly basic anion exchange resin for the second portion, respectively, at an SV of 0.81 and 0.70, and the 6SL adsorbed onto the weakly basic anion exchange resin was eluted as a sodium salt, yielding two solutions C.

Next, as the third step, the two solutions C obtained in the second step were each passed through 11.5 kL of the H⁺ type weakly acidic cation exchange resin PUROLITE (registered trademark) C115E (manufactured by PUROLITE), yielding two solutions D adjusted to a pH range of 2 to 4. Note that solutions C were respectively passed through PUROLITE (registered trademark) C115E at an SV of 1.5 and 1.4 relative to when the resin volume of the third column of PUROLITE (registered trademark) C115E is 1 while cooling to 11.1°C and 15.2°C, respectively.

Then, as the fourth step, the two solutions D obtained in the third step were each passed through 9.5 kL of PUROLITE (registered trademark) A845/1967 (manufactured by PUROLITE), which is an OH⁻ type weakly basic anion exchange resin, while cooling to 8.0°C, yielding 108 kL of eluate (solution E) for the first portion and 80.6 kL of eluate (solution E) for the second portion. Note that solutions D were respectively passed through PUROLITE (registered trademark) A845/1967 at an SV of 1.9 and 1.7 relative to when the resin volume of the fourth column of PUROLITE (registered trademark) A845/1967 is 1.

The pH of the two solutions E obtained in the fourth step was adjusted to 5.5 to 6.1 by adding 0.5 mol/L hydrochloric acid to each. Subsequently, each pH-adjusted solution E was passed through a 1.2 µm in-line filter (MF membrane) and a 0.45 µm in-line filter (MF membrane) in this order, yielding two treated solutions. Next, each of the treated solutions was concentrated until the SL·Na concentration reached 400 g/L, and the two concentrated solutions were mixed to obtain a single concentrated solution.

Next, activated carbon (Taiko (registered trademark) Y) manufactured by FUTAMURA CHEMICAL CO., LTD. was added to this concentrate to adsorb and remove colored components, and the like, in the concentrate. Additionally, immediately prior to proceeding to a filtration process, 0.5 mol/L hydrochloric acid was added to the concentrate containing the activated carbon to adjust the pH to 5.3 to 6.2. This pH-adjusted concentrate was passed through a 0.45 µm in-line filter (MF membrane) to remove fine activated carbon and foreign matter as part of the filtration process, then it was passed through a UF membrane (6000 Da cut-off), thereby removing endotoxins, etc., from the concentrate. Subsequently, the permeate obtained by passing through the UF membrane was concentrated until the SL·Na concentration reached approximately 400 g/L, and a sterilized treated liquid (liquid fermented product) was obtained by passing through a 0.2 µm in-line filter.

Subsequently, as a fifth step, the sterilized treated solution was spray-dried using a spray dryer, yielding spray-dried powder (SD) as the fermented product. This yielded 2099 kg of powder with an SL purity of 88 mass%, a NeuAc content of 0.0170 in terms of ratio to 6SL, and an acetic acid content of 0.00318 in terms of ratio to 6SL, as shown in the "SD" columns of Tables 7 and 8.

Tables 7 and 8 show the results of the analysis of the compositions of solution A, solution E, and SD obtained in Example 4. The various parameters shown in Tables 7 and 8 were calculated using the same methods as those shown in Tables 5 and 6, except that 3SL was replaced with 6SL.

In this powder, glucose and lactose, which are neutral sugars (Z1), had a ratio to 6SL (the ratio (content of component Z1 / content of component X) of the content of each compound of component Z1 relative to the content of component X in the fermented product) below the detection limit.

**[Table 7]**

| Lot | Resin | Sample | 6S L | Liquid Volume | Yield | Moisture Content | SL Purity | Powder Quantity | Acetic Acid | | |
|---|---|---|---|---|---|---|---|---|---|---|---|
| | | | [g/ L] | [kL] | | [%] | [%] | [kg] | [g/L] | [Ratio to 6SL] | Residual Rate |
| Lot 5 | NA | A. Broth | 39. 8 | 83.4 | NA | NA | NA | NA | 3.17 | 0.0796 | NA |
| Lot 5 First Portion | NA | Solution A First Portion | 25. 7 | 60.8 | NA | NA | NA | NA | 2.12 | 0.0826 | NA |
| Lot 5 Second Portion | NA | Solution A Second Portion | 24. 6 | 60.0 | NA | NA | NA | NA | 1.96 | 0.0796 | NA |
| Lot 5 First Portion | 4 Columns | Solution E First Portion | 11. 6 | 108 | 0.8 0 | NA | NA | NA | ND* | ≤ 0.000047 6** (ND*) | 0.0 |
| Lot 5 Second Portion | 4 Columns | Solution E Second Portion | 11. 9 | 80.6 | 0.6 5 | NA | NA | NA | ND* | ≤ 0.000047 6** (ND*) | 0.0 |
| Lot 5 | NA | SD | NA | NA | NA | 7.9 | 88 | 2099 | NA* ** | 0.00318 | NA |

| | | | | | | | | | | | |
|---|---|---|---|---|---|---|---|---|---|---|---|
| NA = not applicable, ND = not detected * Detection limit of 0.000476 g/L for sample solution ** Value when analyzing a sample solution prepared with component X = 6SL at 10 g/L *** 0.0318 when analyzing a sample solution prepared with component X = 6SL at 10 g/L | | | | | | | | | | | |

**[Table 8]**

| Lot | Resin | Sample | Glucose | | Lactose | | NeuAc | |
|---|---|---|---|---|---|---|---|---|
| | | | [g/L] | [Ratio to 6SL] | [g/L] | [Ratio to 6SL] | [g/L] | [Ratio to 6SL] |
| Lot 5 | NA | A. Broth | NA | NA | NA | NA | 11.0 | 0.276 |
| Lot 5 First Portion | NA | Solution A First Portion | NA | NA | NA | NA | 7.52 | 0.292 |
| Lot 5 Second Portion | NA | Solution A Second Portion | NA | NA | NA | NA | 4.83 | 0.196 |
| Lot 5 First Portion | 4 Columns | Solution E First Portion | NA | NA | NA | NA | 0.274 | 0.0236 |
| Lot 5 Second Portion | 4 Columns | Solution E Second Portion | NA | NA | NA | NA | 0.115 | 0.00969 |
| Lot 5 | NA | SD | ND*** | ≤ 0.000467* (ND***) | ND**** | ≤ 0.000451* (ND*) | NA***** | 0.0170 |

| | | | | | | | | |
|---|---|---|---|---|---|---|---|---|
| NA = not applicable, ND = not detected * Value when analyzing a sample solution prepared with component X = 6SL at 1 g/L *** Detection limit of 0.000467 g/L for sample solution **** Detection limit of 0.000451 g/L for sample solution ***** 0.170 g/L when analyzing a sample solution prepared with component X = 6SL at 1 g/L | | | | | | | | |

### [Preparation Example 3] <Fermentative Production of 3SL>

The culture broth was prepared by fermenting 3SL using recombinant Escherichia coli according to the methods described in International Publication No. 2015/037698 and Japanese Patent Application Laid-Open Publication No. 2008-5794, and the culture was heat-treated at 70°C for 40 minutes to inactivate the bacterial cells, and sulfuric acid was added to adjust the pH to 4.3 ± 0.3. Subsequently, the inactivated bacterial cells (biomass) were separated from fermentation solution using a cross-flow filter (0.1 µm filter), and the culture solution (hereinafter referred to as solution A) was obtained.

### [Example 5] <Purification of 3SL via First to Fourth Steps>

As Example 5, the treatments of the first to fourth steps were performed using 0.468 L of solution A obtained in [Preparation Example 3] above.

In the first step, 126 mL of TG-Gel (manufactured by THE DOW CHEMICAL COMPANY), which is an H⁺ type strongly acidic cation exchange resin, was used, solution A was passed through the TG-Gel at an SV of 1.0 at 15.0°C, causing cationic impurities in solution A to be adsorbed onto the strongly acidic cation exchange resin, thereby yielding solution B in which the cationic impurities had been removed from solution A.

Next, 140 mL of PUROLITE (registered trademark) A845/1967 (manufactured by PUROLITE), which is an OH⁻ type weakly basic anion exchange resin, was used as the resin of the second step, and in order to ensure that the anionic impurities and 3SL in solution B were adsorbed by this weakly basic anion exchange resin, solution B was passed through the weakly basic anion exchange resin at 15.0°C and an SV of 0.9. Subsequently, washing was performed by passing 3.5 RV of deionized water at 15.0°C through at an SV of 1.0 to remove neutral substances that do not adsorb onto the weakly basic anion exchange resin from the weakly basic anion exchange resin. Thereafter, the anionic impurities adsorbed onto the weakly basic anion exchange resin were removed by passing 3.0 RV of a 0.005 mol/L NaOH aqueous solution at 15.0°C through the resin at an SV of 1.0. Then a 0.035 mol/L NaOH aqueous solution at 15.0°C was passed through at an SV of 1.0, and the 3SL adsorbed onto the weakly basic anion exchange resin was eluted as a sodium salt, yielding solution C.

Next, as the third step, solution C obtained in the second step was passed through 84 mL of the H⁺ type weakly acidic cation exchange resin PUROLITE (registered trademark) C115E (manufactured by PUROLITE), yielding solution D adjusted to a pH range of 2 to 4. Solution C was passed through PUROLITE (registered trademark) C115E at an SV of 1.6 relative to when the resin volume of the third column of PUROLITE (registered trademark) C115E is 1 while cooling to 15.0°C.

Then, as the fourth step, solution D obtained in the third step was passed through 74 mL of PUROLITE (registered trademark) A845/1967 (manufactured by PUROLITE), which is an OH⁻ type weakly basic anion exchange resin, while cooling to 15.0°C, yielding 1.1050 L of eluate (solution E). Solution D was passed through PUROLITE (registered trademark) A845/1967 at an SV of 1.9 relative to when the resin volume of the fourth column of PUROLITE (registered trademark) A845/1967 is 1, the eluate was collected every 0.6 RV, and the composition of the eluate was analyzed.

The results of the analysis of the composition of solution E obtained in the fourth step are shown in the columns for Example 5 in Tables 10 and 11.

### [Example 6] <Purification of 3SL via First to Fourth Steps>

As Example 6, the treatments of the first to fourth steps were performed using 0.468 L of solution A obtained in [Preparation Example 3] above.

In the first step, 126 mL of TG-Gel (manufactured by THE DOW CHEMICAL COMPANY), which is an H⁺ type strongly acidic cation exchange resin, was used, solution A was passed through the TG-Gel at an SV of 1.0 at 10.0°C, causing cationic impurities in solution A to be adsorbed onto the strongly acidic cation exchange resin, thereby yielding solution B in which the cationic impurities had been removed from solution A.

Next, 140 mL of PUROLITE (registered trademark) A845/1967 (manufactured by PUROLITE), which is an OH⁻ type weakly basic anion exchange resin, was used as the resin of the second step, and in order to ensure that the anionic impurities and 3SL in solution B were adsorbed by this weakly basic anion exchange resin, solution B was passed through the weakly basic anion exchange resin at 10.0°C and an SV of 0.9. Subsequently, washing was performed by passing 3.5 RV of deionized water at 25.0°C through at an SV of 1.0 to remove neutral substances that do not adsorb onto the weakly basic anion exchange resin from the weakly basic anion exchange resin. Thereafter, the anionic impurities adsorbed onto the weakly basic anion exchange resin were removed by passing 3.0 RV of a 0.005 mol/L NaOH aqueous solution at 25.0°C through the resin at an SV of 1.0. Then a 0.035 mol/L NaOH aqueous solution at 25.0°C was passed through at an SV of 1.0, and the 3SL adsorbed onto the weakly basic anion exchange resin was eluted as a sodium salt, yielding solution C.

Next, as the third step, solution C obtained in the second step was passed through 84 mL of the H⁺ type weakly acidic cation exchange resin PUROLITE (registered trademark) C115E (manufactured by PUROLITE), yielding solution D adjusted to a pH range of 2 to 4. Solution C was passed through PUROLITE (registered trademark) C115E at 25.0°C at an SV of 1.6 relative to when the resin volume of the third column of PUROLITE (registered trademark) C115E is 1.

Then, as the fourth step, solution D obtained in the third step was passed through 74 mL of PUROLITE (registered trademark) A845/1967 (manufactured by PUROLITE), which is an OH⁻ type weakly basic anion exchange resin, at 25.0°C, yielding 1.095 L of eluate (solution E). Solution D was passed through PUROLITE (registered trademark) A845/1967 at an SV of 1.9 relative to when the resin volume of the fourth column of PUROLITE (registered trademark) A845/1967 is 1, the eluate was collected every 0.6 RV, and the composition of the eluate was analyzed.

The results of the analysis of the composition of solution E obtained in the fourth step are shown in the columns for Example 6 in Tables 10 and 11.

### [Example 7] <Purification of 3SL via First to Fourth Steps>

As Example 7, the treatments of the first to fourth steps were performed using 0.468 L of solution A obtained in [Preparation Example 3] above.

In the first step, 126 mL of TG-Gel (manufactured by THE DOW CHEMICAL COMPANY), which is an H⁺ type strongly acidic cation exchange resin, was used, solution A was passed through the TG-Gel at an SV of 1.0 at 10.0°C, causing cationic impurities in solution A to be adsorbed onto the strongly acidic cation exchange resin, thereby yielding solution B in which the cationic impurities had been removed from solution A.

Next, 140 mL of PUROLITE (registered trademark) A845/1967 (manufactured by PUROLITE), which is an OH⁻ type weakly basic anion exchange resin, was used as the resin of the second step, and in order to ensure that the anionic impurities and 3SL in solution B were adsorbed by this weakly basic anion exchange resin, solution B was passed through the weakly basic anion exchange resin at 10.0°C and an SV of 0.9. Subsequently, washing was performed by passing 3.5 RV of deionized water at 30.0°C through at an SV of 1.0 to remove neutral substances that do not adsorb onto the weakly basic anion exchange resin from the weakly basic anion exchange resin. Thereafter, the anionic impurities adsorbed onto the weakly basic anion exchange resin were removed by passing 3.0 RV of a 0.005 mol/L NaOH aqueous solution at 30.0°C through the resin at an SV of 1.0. Then a 0.035 mol/L NaOH aqueous solution at 30.0°C was passed through at an SV of 1.0, and the 3SL adsorbed onto the weakly basic anion exchange resin was eluted as a sodium salt, yielding solution C.

Next, as the third step, solution C obtained in the second step was passed through 84 mL of the H⁺ type weakly acidic cation exchange resin PUROLITE (registered trademark) C115E (manufactured by PUROLITE), yielding solution D adjusted to a pH range of 2 to 4. Solution C was passed through PUROLITE (registered trademark) C115E at 30.0°C at an SV of 1.6 relative to when the resin volume of the third column of PUROLITE (registered trademark) C115E is 1.

Then, as the fourth step, solution D obtained in the third step was passed through 74 mL of PUROLITE (registered trademark) A845/1967 (manufactured by PUROLITE), which is an OH⁻ type weakly basic anion exchange resin, at 10.0°C, yielding 1.080 L of eluate (solution E). Solution D was passed through PUROLITE (registered trademark) A845/1967 at an SV of 1.9 relative to when the resin volume of the fourth column of PUROLITE (registered trademark) A845/1967 is 1, the eluate was collected every 0.6 RV, and the composition of the eluate was analyzed.

The results of the analysis of the composition of solution E obtained in the fourth step are shown in the columns for Example 7 in Tables 10 and 11.

### [Example 8] <Purification of 3SL via First to Fourth Steps>

As Example 8, the treatments of the first to fourth steps were performed using 0.468 L of solution A obtained in [Preparation Example 3] above.

In the first step, 126 mL of TG-Gel (manufactured by THE DOW CHEMICAL COMPANY), which is an H⁺ type strongly acidic cation exchange resin, was used, solution A was passed through the TG-Gel at an SV of 1.0 at 10.0°C, causing cationic impurities in solution A to be adsorbed onto the strongly acidic cation exchange resin, thereby yielding solution B in which the cationic impurities had been removed from solution A.

Next, 140 mL of PUROLITE (registered trademark) A845/1967 (manufactured by PUROLITE), which is an OH⁻ type weakly basic anion exchange resin, was used as the resin of the second step, and in order to ensure that the anionic impurities and 3SL in solution B were adsorbed by this weakly basic anion exchange resin, solution B was passed through the weakly basic anion exchange resin at 25.0°C and an SV of 0.9. Subsequently, washing was performed by passing 3.5 RV of deionized water at 25.0°C through at an SV of 1.0 to remove neutral substances that do not adsorb onto the weakly basic anion exchange resin from the weakly basic anion exchange resin. Thereafter, the anionic impurities adsorbed onto the weakly basic anion exchange resin were removed by passing 3.0 RV of a 0.005 mol/L NaOH aqueous solution at 25.0°C through the resin at an SV of 1.0. Then a 0.035 mol/L NaOH aqueous solution at 15.0°C was passed through at an SV of 1.0, and the 3SL adsorbed onto the weakly basic anion exchange resin was eluted as a sodium salt, yielding solution C.

Next, as the third step, solution C obtained in the second step was passed through 84 mL of the H⁺ type weakly acidic cation exchange resin PUROLITE (registered trademark) C115E (manufactured by PUROLITE), yielding solution D adjusted to a pH range of 2 to 4. Solution C was passed through PUROLITE (registered trademark) C115E at an SV of 1.6 relative to when the resin volume of the third column of PUROLITE (registered trademark) C115E is 1 while cooling to 15.0°C.

Then, as the fourth step, solution D obtained in the third step was passed through 74 mL of PUROLITE (registered trademark) A845/1967 (manufactured by PUROLITE), which is an OH⁻ type weakly basic anion exchange resin, while cooling to 15.0°C, yielding 1.150 L of eluate (solution E). Solution D was passed through PUROLITE (registered trademark) A845/1967 at an SV of 1.9 relative to when the resin volume of the fourth column of PUROLITE (registered trademark) A845/1967 is 1, the eluate was collected every 0.6 RV, and the composition of the eluate was analyzed.

The results of the analysis of the composition of solution E obtained in the fourth step are shown in the columns for Example 8 in Tables 10 and 11.

Table 9 shows the liquid flow temperatures for the first to fourth steps in Examples 5 to 8.

In Table 10, the [g/L] value for 3SL is calculated using Equation (1c) of Analysis Example 1-1 above, the [g/L] value for NeuAc is calculated using Equation (4a) of Analysis Example 4 above, the [g/L] value for 3'-sialyllactulose is calculated using Equation (11a) of Analysis Example 5 above, and the [g/L] value for component V is calculated using Equation (12a) of Analysis Example 6 above.

The various parameters shown in Table 10 were calculated using the same methods as those shown in Table 3. The various parameters shown in Table 11 were calculated using the same methods as those shown in Table 6.

In Table 13, the [ratio to 3SL] of acetic acid is a value calculated using Equation (2b) of Analysis Example 2 above; the [ratio to 3SL] of lactose is a value calculated using Equation (3b) of Analysis Example 3 above; the [ratio to 3SL] of NeuAc is a value calculated using Equation (4b) of Analysis Example 4 above; the [ratio to 3SL] of 3'-sialyllactulose is a value calculated using Equation (11b) of Analysis Example 5 above; the [ratio to 3SL] of impurities detected at RT27.0-RT35.0 is a value calculated using Equation (12b) of Analysis Example 6 above; the [ratio to 3SL] of other impurities detected by HPLC is a value calculated using Equation (13b) of Analysis Example 1-2 above; the [ratio to 3SL] of the total value of other impurities detected by HPLC is a value calculated using Equation (13c) of Analysis Example 1-2 above; the [ratio to 3SL] of the total value of impurities and sodium is a value calculated using Equation (14a) of Analysis Example 1-2 above; the total value of impurities and sodium on a wet basis is a value calculated using Equation (14b) of Analysis Example 1-2 above; the 3SL content ratio on a wet basis is a value calculated using Equation (14c) of Analysis Example 1-2 above; and the 3SL content ratio is a value calculated using Equation (14d) of Analysis Example 1-2 above. As shown in Table 13, the 3SL content ratio in Examples 5 to 8 was 80 mass% or higher in each case. In this instance, the "3SL content" in Table 13 refers to the "content ratio of component X per unit of solid matter in the fermented product" when component X is 3SL.

**[Table 9]**

| [°C] | Solution Passed | Example 5 | Example 6 | Example 7 | Example 8 |
|---|---|---|---|---|---|
| First Step | Solution A | 15.0 | 10.0 | 10.0 | 10.0 |
| Second Step (2-1) | Solution B | 15.0 | 10.0 | 10.0 | 25.0 |
| Second Step (2-2) | Deionized water | 15.0 | 25.0 | 30.0 | 25.0 |
| Second Step (2-2) | 0.005 mol/L NaOH | 15.0 | 25.0 | 30.0 | 25.0 |
| Second Step (2-3) | 0.035 mol/L NaOH | 15.0 | 25.0 | 30.0 | 15.0 |
| Third Step | Solution C | 15.0 | 25.0 | 30.0 | 15.0 |
| Fourth Step | Solution D | 15.0 | 25.0 | 10.0 | 15.0 |

**[Table 10]**

| Lot | | Resin | Sample | 3SL | Liquid Volume | Yield | Y (Acetic Acid) | | |
|---|---|---|---|---|---|---|---|---|---|
| | | | | [g/L] | [L] | | [g/L] | [Ratio to 3SL] | Residual Rate |
| Lot 6 | Example 5 | NA | Solution A | 33.7 | 0.468 | NA | 0.847 | 0.0251 | NA |
| | | 4 Columns | Solution E | 14.0 | 1.100 | 0.98 | 0.00774 | 0.000554 | 0.0215 |
| Lot 7 | Example 6 | NA | Solution A | 33.7 | 0.468 | NA | 0.847 | 0.0251 | NA |
| | | 4 Columns | Solution E | 13.9 | 1.095 | 0.97 | 0.00892 | 0.000641 | 0.0246 |
| Lot 8 | Example 7 | NA | Solution A | 33.7 | 0.468 | NA | 0.847 | 0.0251 | NA |
| | | 4 Columns | Solution E | 13.9 | 1.080 | 0.95 | 0.0106 | 0.000765 | 0.0289 |
| Lot 9 | Example 8 | NA | Solution A | 33.7 | 0.468 | NA | 0.847 | 0.0251 | NA |
| | | 4 Columns | Solution E | 13.7 | 1.150 | 1.00 | 0.00727 | 0.000530 | 0.0211 |

| | | | | | | | | | |
|---|---|---|---|---|---|---|---|---|---|
| NA = not applicable | | | | | | | | | |

**[Table 11]**

| Lot | | Resin | Sample | Z1 (Glucose) | | Z1 (Lactose) | | Z2 (NeuAc) | |
|---|---|---|---|---|---|---|---|---|---|
| | | | | [g/L] | [Ratio to 3SL] | [g/L] | [Ratio to 3SL] | [g/L] | [Ratio to 3SL] |
| Lot 6 | Example 5 | NA | Solution A | 0.0719 | 0.00213 | 1.10 | 0.0326 | 1.30 | 0.0386 |
| | | 4 Columns | Solution E | ND | ND | 0.00392 | 0.000281 | 0.0622 | 0.00448 |
| Lot 7 | Example 6 | NA | Solution A | 0.0719 | 0.00213 | 1.10 | 0.0326 | 1.30 | 0.0386 |
| | | 4 Columns | Solution E | ND | ND | 0.00459 | 0.000329 | 0.0309 | 0.00221 |
| Lot 8 | Example 7 | NA | Solution A | 0.0719 | 0.00213 | 1.10 | 0.0326 | 1.30 | 0.0386 |
| | | 4 Columns | Solution E | ND | ND | 0.00600 | 0.000432 | 0.0396 | 0.00285 |
| Lot 9 | Example 8 | NA | Solution A | 0.0719 | 0.00213 | 1.10 | 0.0326 | 1.30 | 0.0386 |
| | | 4 Columns | Solution E | ND | ND | 0.00275 | 0.000200 | 0.0551 | 0.00402 |

| | | | | | | | | | |
|---|---|---|---|---|---|---|---|---|---|
| NA = not applicable, ND = not detected | | | | | | | | | |

**[Table 12]**

| Lot | | Resin | Sample | W (3'-sialyllactulose) | | V (RT27.0-RT35.0) | |
|---|---|---|---|---|---|---|---|
| | | | | [g/L] | [Ratio to 3SL] | [g/L] | [Ratio to 3SL] |
| Lot 6 | Example 5 | NA | Solution A | 0.214 | 0.00636 | 7.72 | 0.229 |
| | | 4 Columns | Solution E | 0.0326 | 0.00233 | 0.589 | 0.0421 |
| Lot 7 | Example 6 | NA | Solution A | 0.214 | 0.00636 | 7.72 | 0.229 |
| | | 4 Columns | Solution E | 0.0527 | 0.00379 | 0.428 | 0.0308 |
| Lot 8 | Example 7 | NA | Solution A | 0.214 | 0.00636 | 7.72 | 0.229 |
| | | 4 Columns | Solution E | 0.0689 | 0.00496 | 0.373 | 0.0268 |
| Lot 9 | Example 8 | NA | Solution A | 0.214 | 0.00636 | 7.72 | 0.229 |
| | | 4 Columns | Solution E | 0.0340 | 0.00248 | 0.426 | 0.0311 |

| | | | | | | | |
|---|---|---|---|---|---|---|---|
| NA = not applicable | | | | | | | |

**[Table 13]**

| Symbol | Calculation Formula | Represents | Example 5 | Example 6 | Example 7 | Example 8 |
|---|---|---|---|---|---|---|
| Y | NA | Acetic Acid [Ratio to 3SL] | 0.000554 | 0.000641 | 0.000765 | 0.000530 |
| Z₁ | NA | Lactose [Ratio to 3SL] | 0.000281 | 0.000329 | 0.000432 | 0.000200 |
| Z₂ | NA | NeuAc [Ratio to 3SL] | 0.00448 | 0.00221 | 0.00285 | 0.00402 |
| W | NA | 3'-sialyllactulose [Ratio to 3SL] | 0.00233 | 0.00379 | 0.00496 | 0.00248 |
| V | NA | Impurities Detected At RT27.0 to RT35.0 [Ratio to 3SL] | 0.0421 | 0.0308 | 0.0268 | 0.0311 |
| U | NA | Other Individual Impurities Detected by HPLC [Ratio to 3SL] | NA | NA | NA | NA |
| U₂ | ${U}_{2} = {\sum }_{i = 1}^{n} Ui$ | Total Value of Other Impurities Detected by HPLC [Ratio to 3SL] | 0.0332 | 0.0303 | 0.0311 | 0.0301 |
| M₁ | NA | Sodium [Ratio to 3SL] | 0.0339* | 0.0339* | 0.0339* | 0.0339* |
| U₃ | NA | Moisture Content [mass%] | 7.95** | 7.95** | 7.95** | 7.95** |
| I₁ | Y + Z₁ + Z₂ + W + V + U₂ + M₁ | Total Value of Impurities and sodium [Ratio to 3SL] | 0.117 | 0.102 | 0.101 | 0.102 |
| I₂ | I₁ × (100 - U₃) / 100 | Total Value of Wet-Basis Impurities and sodium [-] | 0.108 | 0.0939 | 0.0928 | 0.0942 |
| X₁ | 100 - I₂ × 100 - U₃ | Wet-Basis 3SL Content Ratio [mass%] | 81.3 | 82.7 | 82.8 | 82.6 |
| X₂ | X₁ / (100 - U₃) × 100 | 3SL Content Ratio [mass%] | 88.3 | 89.8 | 89.9 | 89.8 |

| | | | | | | |
|---|---|---|---|---|---|---|
| NA = not applicable *: Using a representative example of sodium measured when solution E was powderized **: Using a representative example of moisture content measured when solution E was powderized | | | | | | |

Thus, it was found that treating a solution containing sialyllactose with the first to fourth steps yields solution E, which contains a high concentration of sialyllactose and has a low acetic acid concentration. Furthermore, it was found that powderizing this solution in the fifth step yields a fermented product which contains a high concentration of sialyllactose and has a low acetic acid concentration. Such a fermented product with a low acetic acid concentration can be considered highly safe for the human body.

It is clear that a person skilled in the art could conceive of various modifications or alterations within the scope described in the claims, and it is understood that such examples would also naturally fall within the technical scope of the present invention. Furthermore, the various constituent components of the above embodiments may be combined arbitrarily within a range that does not deviate from the spirit of the invention.

The present application is based on an international application (PCT/JP2023/044738) filed on December 13, 2023, the content of which is incorporated herein by reference.

## Claims

1. A fermented product containing component X, component Y, and component Z,
wherein: component X is a sialic acid or a sialylated oligosaccharide;
the content of component X per unit of solid content of the fermented product is 80 mass% or more;
component Y is acetic acid;
the ratio (content of component Y / content of component X) of the content of component Y relative to the content of component X in the fermented product is 0.010 or less on a mass basis;
component Z is at least one of component Z1 and component Z2, and component Z is a component other than component X; and
component Z1 is a neutral sugar and component Z2 is an acidic sugar.

2. The fermented product as claimed in claim 1, wherein component X is N-acetylneuraminic acid.

3. The fermented product as claimed in claim 1, wherein component X is at least one compound selected from the group consisting of sialyllactose, sialyllacto-N-biose (SLNB), sialyllacto-N-acetylglucosamine (SLNac), sialyllacto-N-tetraose (LST), and disialyllacto-N-tetraose (DSLNT).

4. The fermented product as claimed in claim 2, wherein component Z2 is at least one compound selected from the group consisting of glucuronic acid, mannuronic acid, and galacturonic acid.

5. The fermented product as claimed in claim 3, wherein component Z2 is at least one compound selected from the group consisting of N-acetylneuraminic acid, glucuronic acid, mannuronic acid, and galacturonic acid.

6. The fermented product as claimed in either of claims 3 and 5, further comprising component W, wherein component W is an isomer of component X, and component W is a different substance from component Z2.

7. The fermented product as claimed in any one of claims 1-6, further comprising component V, wherein component V is a substance that is detected at RT27.0-35.0 when the fermented product is measured with HPLC-CAD, and component V is a different substance from component Z2.

8. The fermented product as claimed in either of claims 4 and 5, wherein the ratio (content of component Z2 / content of component X) of the content of each compound of component Z2 relative to the content of component X in the fermented product is 0.02 or less on a mass basis.

9. The fermented product as claimed in any one of claims 1-8, wherein component Z1 is at least one compound of glucose and lactose.

10. The fermented product as claimed in claim 9, wherein the ratio (content of component Z1 / content of component X) of the content of each compound of component Z1 relative to the content of component X in the fermented product is 0.03 or less on a mass basis.

11. The fermented product as claimed in claim 6, wherein the ratio (content of component W / content of component X) of the content of each compound of component W relative to the content of component X in the fermented product is 0.0035 or less on a mass basis.

12. The fermented product as claimed in claim 7, wherein the ratio (content of component V / content of component X) of the total content of component V relative to the content of component X in the fermented product is 0.040 or less on a mass basis.

13. A method for producing a fermented product, the method comprising 1) to 4) below in this order,
1) treating solution A, which contains components X, Y, and Z, with a strongly acidic cation exchange resin to obtain solution B,
2) treating solution B with a weakly basic anion exchange resin to obtain solution C,
3) adjusting the pH of solution C to obtain solution D, and
4) treating solution D with a weakly basic anion exchange resin to obtain solution E,
wherein: component X is a sialic acid or a sialylated oligosaccharide;
component Y is acetic acid;
component Z is at least one of component Z1 and component Z2, and component Z is a component other than component X; and
component Z1 is a neutral sugar and component Z2 is an acidic sugar;
the ratio (total mass of component X in solution E / total mass of component X in solution A) of the total mass of component X in solution E relative to the total mass of component X in solution A is 0.5 or more; and
the ratio (content of component Y / content of component X) of the content of component Y relative to the content of component X in solution E is 0.010 or less on a mass basis.

14. The method for producing a fermented product as claimed in claim 13, further comprising 5) below,
5) powderizing solution E,
wherein: the fermented product contains component X, component Y, and component Z;
the content of component X per unit of solid content of the fermented product is 80 mass% or more; and
the ratio (content of component Y / content of component X) of the content of component Y relative to the content of component X in the fermented product is 0.010 or less on a mass basis.

15. The method for producing a fermented product as claimed in either of claims 13 and 14, wherein the ratio (total mass of component Y in solution E / total mass of component Y in solution A) of the total mass of component Y in solution E relative to the total mass of component Y in solution A is 0.5 or more.

16. The method for producing a fermented product as claimed in any one of claims 13-15, wherein component X is N-acetylneuraminic acid.

17. The method for producing a fermented product as claimed in any one of claims 13-15, wherein component X is at least one compound selected from the group consisting of sialyllactose, sialyllacto-N-biose (SLNB), sialyllacto-N-acetylglucosamine (SLNac), sialyllacto-N-tetraose (LST), and disialyllacto-N-tetraose (DSLNT).

18. The method for producing a fermented product as claimed in claim 16, wherein component Z2 is at least one compound selected from the group consisting of glucuronic acid, mannuronic acid, and galacturonic acid.

19. The method for producing a fermented product as claimed in claim 17, wherein component Z2 is at least one compound selected from the group consisting of N-acetylneuraminic acid, glucuronic acid, mannuronic acid, and galacturonic acid.

20. The method for producing a fermented product as claimed in either of claims 17 and 19, wherein the fermented product further comprises component W, component W is an isomer of component X, and component W is a different substance from component Z2.

21. The method for producing a fermented product as claimed in any one of claims 13-20, wherein the fermented product further comprises component V, component V is a substance that is detected at RT27.0-35.0 when the fermented product is measured with HPLC-CAD, and component V is a different substance from component Z2.

22. The method for producing a fermented product as claimed in either of claims 18 and 19, wherein the ratio (content of component Z2 / content of component X) of the content of each compound of component Z2 relative to the content of component X in the fermented product is 0.02 or less on a mass basis.

23. The method for producing a fermented product as claimed in any one of claims 13-22, wherein component Z1 is at least one compound of glucose and lactose.

24. The method for producing a fermented product as claimed in claim 23, wherein the ratio (content of component Z1 / content of component X) of the content of each compound of component Z1 relative to the content of component X in the fermented product is 0.03 or less on a mass basis.

25. The method for producing a fermented product as claimed in claim 13,
wherein 2) above comprises 2-1) to 2-3) below in this order.
2-1) passing solution B through a weakly basic anion exchange resin to remove at least a portion of neutral impurities that do not adsorb onto the weakly basic anion exchange resin,
2-2) eluting at least a portion of the impurities remaining after 2-1) from the weakly basic anion exchange resin, and
2-3) separately eluting acidic compounds containing component X and the impurities remaining after 2-2) from the weakly basic anion exchange resin

26. The method for producing a fermented product as claimed in claim 25, wherein the fluid temperature of the weakly basic anion exchange resin in 2-2) above is 20°C or higher.

27. The method for producing a fermented product as claimed in either of claims 25 and 26, wherein the fluid temperature of the weakly basic anion exchange resin in 2-3) above is 20°C or lower.

28. The method for producing a fermented product as claimed in any one of claims 25-27, wherein the fluid temperature of the weakly basic anion exchange resin in 2-1) above is 15°C or lower.

29. The method for producing a fermented product as claimed in either of claims 25 and 26, wherein: the fermented product further comprises component V, component V is a substance that is detected at RT27.0-35.0 when the fermented product is measured with HPLC-CAD, and component V is a different substance from component Z2; and
the ratio (content of component V / content of component X) of the total content of component V relative to the content of component X in the fermented product is 0.040 or less on a mass basis.

30. The method for producing a fermented product as claimed in either of claims 25 and 27, wherein: the fermented product further comprises component W, component W is an isomer of component X, and component W is a different substance from component Z2; and
the ratio (content of component W / content of component X) of the content of each compound of component W relative to the content of component X in the fermented product is 0.0035 or less on a mass basis.

31. The method for producing a fermented product as claimed in claim 25, wherein the fluid temperature of the weakly basic anion exchange resin in the step of 2-1) above is 5-15°C, the fluid temperature of the weakly basic anion exchange resin in the step of 2-2) above is 20-25°C, and the fluid temperature of the weakly basic anion exchange resin in the step of 2-3) above is 8-20°C.
